# EUROPEAN PATENT SPECIFICATION

(11) **EP 3 851 158 B1**
(45) Date of publication and mention of the grant of the patent: **17.12.2025**
(21) Application number: 19861123.8
(22) Date of filing: 10.09.2019
(51) Int. Cl.: A61N 1/36, A61N 1/04, A61N 1/08

(54) **ELECTRICAL STIMULATOR**
ELEKTRISCHER STIMULATOR
STIMULATEUR ÉLECTRIQUE

(30) Priority: 11.09.2018 JP 2018169445; 20.08.2019 JP 2019150576
(43) Date of publication of application: 21.07.2021
(73) Proprietor: AI Silk Corporation, Sendai-shi, Miyagi 980-8579 (JP)
(72) Inventor: OKANO Hideo, Sendai-shi, Miyagi 980-8579 (JP); MEGURO Shinya, Sendai-shi, Miyagi 980-8579 (JP)
(74) Representative: TBK
(86) International application number: PCT/JP2019/035573
(87) International publication number: WO 2020/054727

(56) References cited:
- WO-A1-2016/013067
- JP-A- 2002 534 231
- JP-A- 2012 531 990
- JP-A- 2015 077 226
- JP-A- 2015 077 226
- JP-A- 2018 015 435
- JP-A- 2018 015 638
- JP-A- H08 164 213
- JP-A- H09 154 956
- US-A1- 2012 116 477
- US-A1- 2018 036 531
- US-B1- 9 339 642

## Description

### Technical Field

The present invention relates to an electrical stimulator that applies electrical stimulation to a body by use of an electrode group having three or more electrodes.

### Background Art

An electrical stimulator that applies electrical stimulation to a body while bringing an electrode into contact with a predetermined position of the body so as to conduct muscle training or the like has been known in recent years. For example, a device that gives energy to a specific pair of electrodes and that applies electrical stimulation between these electrodes can be mentioned as the electrical stimulator formed as above. For example, document JP 2012 - 531 990 A discloses a device that includes electrode segments A1 and A2, which are applied to a lower lumbar region of a patient's body, and electrodes B and C, which are respectively applied to mutually-facing flanks of the patient's body, and that alternately gives a first group of muscle-stimulation current pulses flowing between the electrode B and the electrode C and a second group of muscle-stimulation current pulses flowing between the electrode segments A1, A2 and the electrodes B, C.

### Citation List

Document JP 2018 - 015 638 A discloses a muscle electrostimulation device which is attached to the abdomen of a person to apply electrostimulation to the abdomen from an electrode unit. The electrode unit includes: a first electrode group that extends from a main body so as to, when the device is attached to the abdomen, be disposed parallel to the height direction of the person and on a right hand side of the person with respect to a center line passing through a center of the main body; and a second electrode group that extends from the main body so as to be disposed on a left hand side of the person with respect to the center line. The first electrode group and the second electrode group can be electronically connected via the person, and the first electrode group and the second electrode group include three or more electrodes respectively. In the first electrode group and the second electrode group, the respective electrodes are arranged into a circular arc shape that is curved so as to be convex in an opposite direction of the main body.

Document JP 2015 - 077 226 A discloses a wearable electrode which includes: a bioelectrode; and equipment that holds the bioelectrode by having a base band part laterally making a round of the chest and a diagonal band part obliquely elongated from a front chest arrangement position on one horizontal side of the base band part and connected to a back part arrangement position on the one horizontal side of the base band part via the shoulder on the opposite horizontal side thereof.

### Summary of Invention

### Technical Problem

However, in the device of document JP 2012 - 531 990 A, energy is given between the electrode segments A1, A2 and the electrodes B, C, and therefore the electrodes that apply stimulation are in a multielectrode-multielectrode relationship, and a problem has resided in the fact that it is difficult to understand where defects have been caused, and it is impossible to apply targeted stimulation if an insufficient state occurs in a wiring state or in a contact state between the electrodes and the body in part of the electrodes.

Additionally, a problem has resided in the fact that, if an insufficient state occurs in a wiring state or in a contact state between the electrodes and the body in part of the electrodes, unintentional strong stimulation will be applied to another part of the electrodes. For example, in the device of document JP 2012 - 531 990 A, if debonding occurs in the electrode B when energy is given between the electrode segments A1, A2 and the electrodes B, C, stimulation will concentrate at the electrode C, and unintentional strong stimulation will be applied thereto. Hence, there is a concern that a user will receive an uncomfortable sensation while feeling strong stimulation, or will be surprised at strong stimulation, and, probably, will reflexively move his/her body, and might receive an injury. Additionally, there is a concern that the user will suffer a low temperature burn because of the concentration of an electric current.

Additionally, a problem has resided in the fact that the strength of stimulation felt by the human body varies in each electrode because of variation in a wiring state or in a contact state between an electrode and the body when energy is given between a multielectrode and a multielectrode.

Based on these problems, the present invention has been made, and a first object of the present invention is to provide an electrical stimulator that is capable of easily detecting electrical connection defects.

A second object of the present invention is to provide an electrical stimulator that is capable of restraining the occurrence of unintentional strong stimulation.

A third object of the present invention is to provide an electrical stimulator that is capable of applying less-variable stimulation in each electrode.

### Solution to Problem

These object are achieved by an electrical stimulator according to claim 1. Advantageous further developments are as set forth in the dependent claims.

An electrical stimulator according to one aspect applies electrical stimulation to a user, and includes an electrode group that has three or more electrodes that are disposed so as to come into contact with the user, a driving means that gives energy to the electrode group, under a condition that one or more electrodes of the electrode group are each set as a first polarity and that one or more other electrodes are each set as a second polarity, while performing switching among at least either the electrodes each of which is set as the first polarity or the electrodes each of which is set as the second polarity, and a connection-defect detection means that detects an electrical connection defect by detecting whether electrical stimulation has been applied to the user when energy is given to the electrode group, and, in the electrical stimulator, each of the electrodes consists of a conductor in which an electroconductive polymer adheres to a base made of fiber.

For example, the driving means may give energy to the electrode group, under a condition that one electrode of the electrode group is set as the first polarity and that plurality of remaining electrodes are each set as the second polarity, while performing switching among electrodes each of which is set as the first polarity in predetermined order, or may give energy to the electrode group, under a condition that one electrode of the electrode group is set as the first polarity and that one other electrode is set as the second polarity, while performing switching among at least either the electrodes each of which is set as the first polarity or the electrodes each of which is set as the second polarity in turn, or may give energy to the electrode group, under a condition that one or more electrodes of the electrode group are each set as the first polarity and that one or more other electrodes are each set as the second polarity, while selectively performing switching among the electrodes each of which is set as the first polarity and as the second polarity in accordance with a muscle action pattern.

### Advantageous Effects of Invention

According to the present invention, energy is given to the electrode group while performing switching among the electrodes, which are each set as the first polarity and as the second polarity, of the electrode group having three or more electrodes, and an electrical connection defect is detected by detecting whether electrical stimulation has been applied to the user when energy is given to the electrode group, and therefore it is possible to easily detect a defect while applying electrical stimulation. Therefore, when peel-off of the electrode or a contact defect has occurred, it is possible to quickly correct such failures during use.

Additionally, if one electrode of the electrode group is set as the first polarity, and the remaining plurality of electrodes are each set as the second polarity, it is possible to allow an electric current to flow between the single electrode of the first polarity and the remaining plurality of electrodes each of which is the second polarity, to allow stimulation to concentrate at the single electrode of the first polarity, and to reduce stimulation in the plurality of electrodes each of which is the second polarity. Depending on circumstances, it is possible to eliminate the stimulation of the electrode of the second polarity or to greatly weaken the stimulation thereof. Therefore, it is possible to restrain the application of unintentional strong stimulation to the other electrodes each of which has the second polarity, for example, even if wiring disconnection has occurred or even if a contact state with the body becomes worse because of peel-off or the like in any one of the plurality of electrodes each of which has the second polarity. Therefore, it is possible to restrain the user from receiving an uncomfortable sensation while feeling strong stimulation or to restrain the user from being surprised at strong stimulation and from reflexively moving the user's body, and it is possible to prevent low temperature burn that is caused by the concentration of an electric current. This effect is effective particularly when high-frequency electrical stimulation is applied.

Additionally, if stimulation is concentrated at one electrode of the first polarity by giving energy under the condition that the number of first polarities is one and that the number of second polarities is two or more, and if switching is performed among the electrodes of the first polarity in predetermined order in the electrode group, it is possible to apply less-variable strong stimulation in turn in all electrodes. Additionally, it is possible to concentrate stimulation at the single electrode of the first polarity, and therefore it is possible to apply strong stimulation with lower energy than in the past.

Still additionally, if energy is given to the electrode group under the condition that one electrode of the electrode group is set as the first polarity and that the remaining plurality of electrodes are each set as the second polarity, it is possible to easily detect an electrical connection defect by use of the fact that electrical stimulation is not applied to the user if there is an electrical connection defect in the electrode set as the first polarity.

Still additionally, at least, if the first electrode is disposed correspondingly to right rectus abdominis muscles, the second electrode is disposed correspondingly to left rectus abdominis muscles, the third electrode is disposed correspondingly to right-hand oblique abdominal muscles, and the fourth electrode is disposed correspondingly to left-hand oblique abdominal muscles, and if switching among the electrodes each of which is set as the first polarity is performed in predetermined order so that the first electrode and the second electrode do not consecutively become the first polarity, it is possible to restrain electrical stimulation from being consecutively applied to muscles near the first and second electrodes that are disposed close to each other and to restrain a relaxation operation from becoming insufficient. Therefore, it is possible to more effectively perform a relaxation operation in the whole of the range in which the electrode group is disposed, and it is possible to restrain early muscle fatigue from being caused or to restrain the user from receiving an uncomfortable sensation.

### Brief Description of Drawings

[FIG. 1] FIG. 1 is a view representing a configuration of an electrical stimulator according to a first embodiment of the present invention.
[FIG. 2] FIG. 2 is a view representing a circuit configuration of a driving means shown in FIG. 1.
[FIG. 3] FIG. 3 is a view representing a circuit configuration of a connection-defect detecting means shown in FIG. 1.
[FIG. 4] FIG. 4 is another view representing the circuit configuration of the connection-defect detecting means shown in FIG. 1.
[FIG. 5] FIG. 5 is still another view representing the circuit configuration of the connection-defect detecting means shown in FIG. 1.
[FIG. 6] FIG. 6 is a view to describe the operation of the electrical stimulator shown in FIG. 1.
[FIG. 7] FIG. 7 is another view to describe the operation of the electrical stimulator shown in FIG. 1.
[FIG. 8] FIG. 8 is a view to describe the operation of a conventional electrical stimulator.
[FIG. 9] FIG. 9 is a view representing a configuration of an electrical stimulator according to a second embodiment of the present invention.
[FIG. 10] FIG. 10 is a view representing the order of a first electrode when energy is given to an electrode group by a driving means shown in FIG. 9.
[FIG. 11] FIG. 11 is a view representing a configuration of an electrical stimulator according to a third embodiment of the present invention.
[FIG. 12] FIG. 12 is a view representing a circuit configuration of a driving means shown in FIG. 11.
[FIG. 13] FIG. 13 is a view representing a circuit configuration of a connection-defect detecting means shown in FIG. 11.
[FIG. 14] FIG. 14 is another view representing the circuit configuration of the connection-defect detecting means shown in FIG. 11.
[FIG. 15] FIG. 15 is still another view representing the circuit configuration of the connection-defect detecting means shown in FIG. 11.
[FIG. 16] FIG. 16 is a view representing a configuration of an electrical stimulator according to a fourth embodiment of the present invention.
[FIG. 17] FIG. 17 is a view representing a configuration of an electrical stimulator according to a fifth embodiment of the present invention.
[FIG. 18] FIG. 18 is a view to describe the operation of the electrical stimulator shown in FIG. 17.
[FIG. 19] FIG. 19 is another view to describe the operation of the electrical stimulator shown in FIG. 17.
[FIG. 20] FIG. 20 is a view representing a configuration of an electrical stimulator according to a sixth embodiment of the present invention.

### Description of Embodiments

Embodiments of the present invention will be hereinafter described in detail with reference to the drawings.

### (First Embodiment)

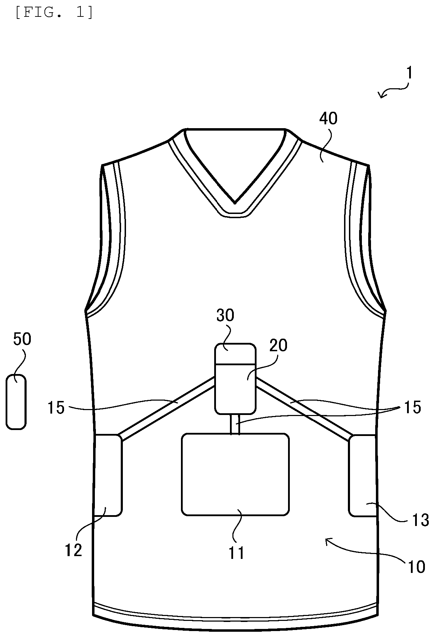

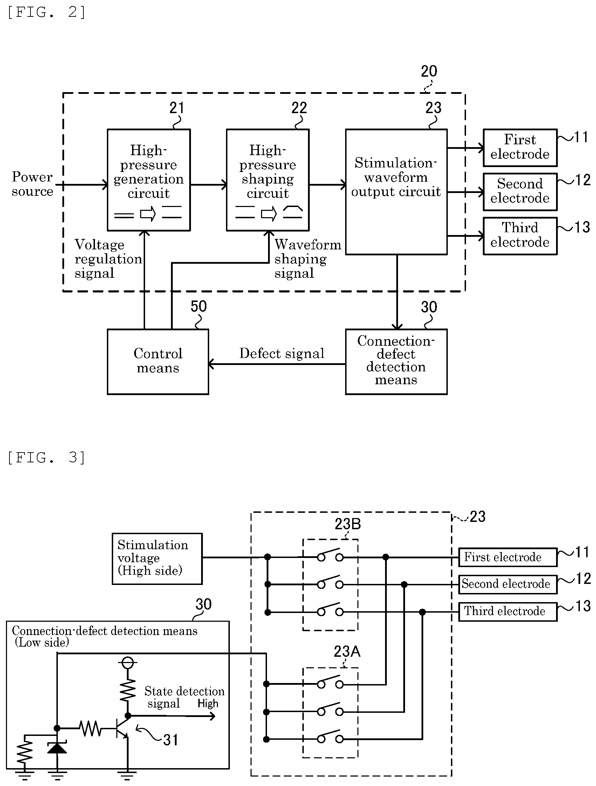

FIG. 1 represents a configuration of an electrical stimulator 1 according to a first embodiment of the present invention. FIG. 2 to FIG. 5 each represent a circuit configuration of the electrical stimulator 1. For example, this electrical stimulator 1 applies electrical stimulation to a user, and includes an electrode group 10 having three or more electrodes, a driving means 20 that gives energy to the electrode group 10, a connection-defect detection means 30 that detects electrical connection defects, a garment 40 on which the electrode group 10 is disposed at a predetermined position, and a control means 50 that controls the driving means 20.

The electrode group 10 is, for example, brought into contact with a user's body and is disposed at a predetermined position. In the present embodiment, for example, the electrode group 10 is disposed at a trunk, such as an abdominal region, and has at least three electrodes. In detail, the electrode group 10 has a first electrode 11 disposed correspondingly to rectus abdominis muscles, a second electrode 12 disposed correspondingly to right-hand oblique abdominal muscles, and a third electrode 13 disposed correspondingly to left-hand oblique abdominal muscles.

Under the condition that one or more electrodes of the electrode group 10 are each set as the first polarity and that one or more other electrodes are each set as the second polarity, the driving means 20 gives energy to the electrode group 10 while performing switching among at least either the electrodes set as the first polarity or the electrodes set as the second polarity. In detail, the driving means 20 is configured to give energy to the electrode group 10, under the condition that, for example, one electrode of the electrode group 10 is set as the first polarity and that the remaining plurality of electrodes are each set as the second polarity, while performing switching among the electrodes that are each set as the first polarity in predetermined order. This makes it possible to concentrate stimulation at the single electrode having the first polarity, and to disperse stimulation with respect to the plurality of electrodes each of which has the second polarity, and to apply strong stimulation in the electrode having the first polarity. The first polarity and the second polarity differ from each other in polarity, and, for example, the first polarity is a positive electrode whereas the second polarity is a negative electrode, or, the first polarity is a negative electrode whereas the second polarity is a positive electrode. Switching among the electrodes each of which is set as the first polarity may be repeatedly performed in the following order, i.e., for example, in order of the first electrode 11, the second electrode 12, and the third electrode 13, or in order of the first electrode 11, the third electrode 13, and the second electrode 12, or in order of the first electrode 11, the second electrode 12, the first electrode 11, and the third electrode 13.

The driving means 20 is, for example, arranged on the garment 40, and is connected to the electrode group 10, to the connection-defect detection means 30, and to the control means 50. With respect to a circuit configuration of the driving means 20, the driving means 20 has, for example, a high-pressure generation circuit 21 that is connected to a power source (not shown) and that generates a high-pressure DC voltage, a high-pressure shaping circuit 22 that is connected to the high-pressure generation circuit 21 and that shapes a waveform, and a stimulation-waveform output circuit 23 that is connected to the high-pressure shaping circuit 22 and to each electrode of the electrode group 10 and that outputs a stimulation waveform to each electrode of the electrode group 10.

Preferably, the high-pressure generation circuit 21 is configured to be capable of changing a to-be-generated voltage value by the control means 50 although the to-be-generated voltage value may be fixed at a constant value. Preferably, the high-pressure shaping circuit 22 is configured to be capable of changing a waveform by the control means 50 although the waveform that has been shaped may be fixed in a constant waveform. The stimulation-waveform output circuit 23 is configured to apply a voltage between an electrode and an electrode, for example, under the condition that one electrode of the electrode group 10 is set as the first polarity, and that the remaining electrodes are each set as the second polarity, while performing switching among the electrodes that are each set as the first polarity in turn.

The connection-defect detection means 30 detects electrical connection defects by detecting whether electrical stimulation has been applied to a user when energy is given to the electrode group 10. For example, wiring breakage, peel-off from a user's body, and contact failure in each electrode of the electrode group 10 can be mentioned as the electrical connection defects. The connection-defect detection means 30 is configured to detect that an electrode, which has been set as the first polarity, has an electrical connection defect, for example, if electrical stimulation is not applied to the user when energy is given to the electrode group 10. This uses the fact that electrical stimulation is not applied to the user if there are electrical connection defects in the electrode that has been set as the first polarity, because one electrode of the electrode group 10 is set as the first polarity, whereas the remaining plurality of electrodes are each set as the second polarity, and energy is given to the electrode group 10 while performing switching among the electrodes each of which is set as the first polarity in predetermined order in the driving means 20.

A configuration shown in, for example, FIG. 3 can be mentioned as the circuit configuration of the connection-defect detection means 30. For example, the connection-defect detection means 30 has a grounded-emitter NPN bipolar transistor 31, and a Low-side switch group 23A corresponding to each electrode of the stimulation-waveform output circuit 23 is connected to a base of the NPN bipolar transistor 31. Hence, in the connection-defect detection means 30, the Low-side switch group 23A and a High-side switch group 23B corresponding to each electrode of the stimulation-waveform output circuit 23 are all brought into an open state as shown in, for example, FIG. 3, and, when energy is not given to the electrode group 10, a collector of the NPN bipolar transistor 31 becomes High, and, as a result, it is possible to obtain a High state detection signal from the collector.

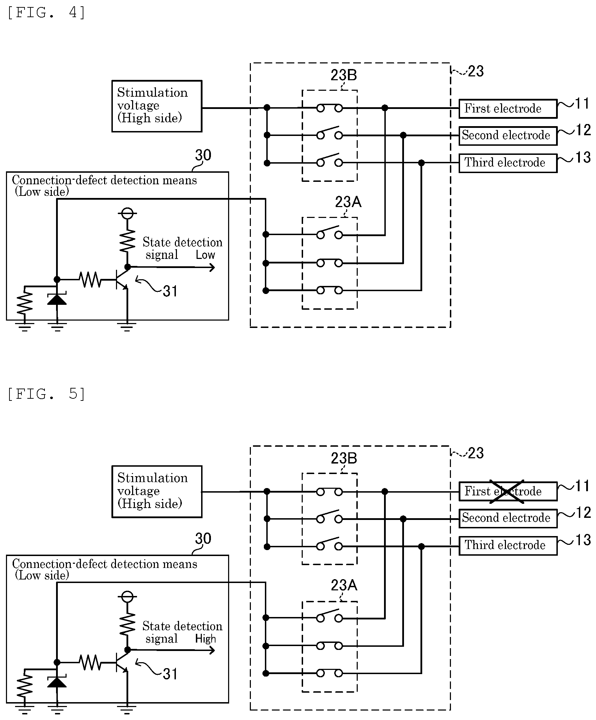

Additionally, as shown in FIG. 4, switches corresponding to the first electrode 11 are brought into a closed state, whereas the other switches are brought into an open state in the High-side switch group 23B of the stimulation-waveform output circuit 23, and switches corresponding to the first electrode 11 are brought into an open state, whereas the other switches are brought into a closed state in the Low-side switch group 23A, and, when energy is given to the electrode group 10 under the condition that the first electrode 11 is set as the first polarity, and the other electrodes, i.e., the second and third electrodes 12 and 13 are each set as the second polarity, an electric current flows through the user's body between the first electrode 11 and the second and third electrodes 12 and 13, and electric potential is applied to the base of the NPN bipolar transistor 31, and a collector current flows, and the collector becomes Low, and, as a result, it is possible to obtain a Low state detection signal from the collector. In other words, the state detection signal of the collector becomes a signal that differs from the signal obtained when energy is not given to the electrode group 10.

However, even when energy is given to the electrode group 10 under the condition that the first electrode 11 is set as the first polarity and that the other electrodes, i.e., the second and third electrodes 12, 13 are each set as the second polarity in the same way as in FIG. 4, an electric current does not flow between the first electrode 11 and the second and third electrodes 12, 13, and electrical stimulation is not applied to the user as shown in FIG. 5 if an electrical connection defect occurs in the first electrode 11 because of peel-off or the like, and therefore electric potential is not applied to the base of the NPN bipolar transistor 31, and the collector becomes High, and a High state detection signal is obtained from the collector. In other words, the state detection signal of the collector becomes the same signal as when energy is not given to the electrode group 10. Therefore, the state signal of the collector depends on whether electrical stimulation has been applied to the user when energy is given to the electrode group 10.

Hence, the connection-defect detection means 30 is configured to detect whether electrical stimulation has been applied to the user when energy is given to the electrode group 10 by detecting the state detection signal of the collector of the NPN bipolar transistor 31, and to detect that an electrical connection defect has occurred in the electrode that has been set as the first polarity when electrical stimulation is not applied to the user.

In the above description, a case has been described in which a switch corresponding to the electrode set as the first polarity is brought into a closed state in the High-side switch group 23B of the stimulation-waveform output circuit 23, whereas a switch corresponding to the electrode set as the first polarity is brought into an open state in the Low-side switch group 23A, and yet the same applies to a case in which a switch corresponding to the electrode set as the first polarity is brought into a closed state in the Low-side switch group 23A, whereas a switch corresponding to the electrode set as the first polarity is brought into an open state in the High-side switch group 23B.

The connection-defect detection means 30 is, for example, arranged on the garment 40, and is connected to the driving means 20 and to the control means 50. Preferably, the connection-defect detection means 30 is configured, for example, to output a defect signal to the control means 50 when an electrical connection defect is detected. Preferably, the control means 50 is configured to display an electrode having an electrical connection defect when a defect signal is received from the connection-defect detection means 30.

Any type of clothing item may be used as the garment 40 as long as it is worn on a user's body. For example, the garment 40 may be a closing item such as a running shirt or a T-shirt as shown in FIG. 1 with which the trunk is covered, and may be a strip-shaped or belt-shaped item that is worn around a user's abdominal region. Although a sleeveless clothing item is shown in FIG. 1, a sleeved clothing item may also be used, and may have a part with which not only an upper half of the body but also a lower half of the body is covered. Additionally, preferably, the garment 40 is made of a stretchy material. The reason is that such a stretchy material enables the electrode group 10 to fit close to the body.

The control means 50 is, for example, configured to be capable of performing control while being kept on hand without being arranged on the garment 40. The connection between the control means 50 and the driving means 20 and connection-defect detection means 30 may be wired or may be wireless. Additionally, preferably, the control means 50 is provided with, for example, a switch that issues a drive command and a stop command to the driving means 20.

Each electrode of the electrode group 10 can be made of, for example, a conductor in which an electroconductive polymer adheres to a base. Any kind of materials of which the base is made may be used, and it is preferable to use fiber, for example, that includes at least one kind of natural fiber, such as silk or cotton, and artificial fiber, such as synthetic fiber. According to the invention, poly-3,4-ethylenedioxythiophene (PEDOT) is used as the electroconductive polymer. The conductor can be obtained, for example, by polymerizing monomers of the electroconductive polymer adhering to the base by use of an oxidizer. In that case, either a dopant that allows the electroconductive polymer to exhibit electroconductivity or a thickening agent may be allowed to adhere to the base together with the monomers of the electroconductive polymer.

Preferably, for example, iron salt can be mentioned as the oxidizer. Preferably, for example, p-toluenesulfonic acid can be mentioned as the dopant, and it is more preferable to use iron salt of p-toluenesulfonic acid (pTS) because that can be allowed to function as the oxidizer and as the dopant. Besides, acetonitrile, trifluoroacetic acid, etc., can be mentioned as the dopant. The thickening agent is used to reduce the bleeding of an electroconductive polymer and to accelerate the polymerization reaction of monomers. Preferably, an agent that does not react to the polymerization reaction of the electroconductive polymer is used as the thickening agent, and, preferably, glycerol, polyethylene glycol, gelatin, or polysaccharides can be mentioned as examples of the thickening agent.

Each electrode of the electrode group 10 may be, for example, formed directly on the garment 40 by using fiber, of which the garment 40 is made, as the base and by allowing an electroconductive polymer to adhere thereto by printing or the like, and may be arranged by bonding or sewing a conductor, which is formed by allowing an electroconductive polymer to adhere to a base prepared independently of the garment 40, onto the garment 40. Lead wires 15 by which each electrode of the electrode group 10 and the driving means 20 are connected together can also be formed in the same way as the electrode group 10.

This electrical stimulator 1 is used as follows. First, a user wears the garment 40 on a user's body to dispose each electrode of the electrode group 10 at a predetermined position of the body. Thereafter, the control means 50 issues a drive command to the driving means 20. In the driving means 20, for example, one electrode of the electrode group 10 is set as the first polarity, and the remaining plurality of electrodes are each set as the second polarity, and energy is given to the electrode group 10 while performing switching among the electrodes each of which is set as the first polarity in predetermined order. Hence, an electric current flows between the single electrode of the first polarity and the remaining plurality of electrodes of the second polarity. Therefore, stimulation concentrates at the single electrode of the first polarity, and, in the electrodes of the first polarity, less-variable strong stimulation is applied in turn.

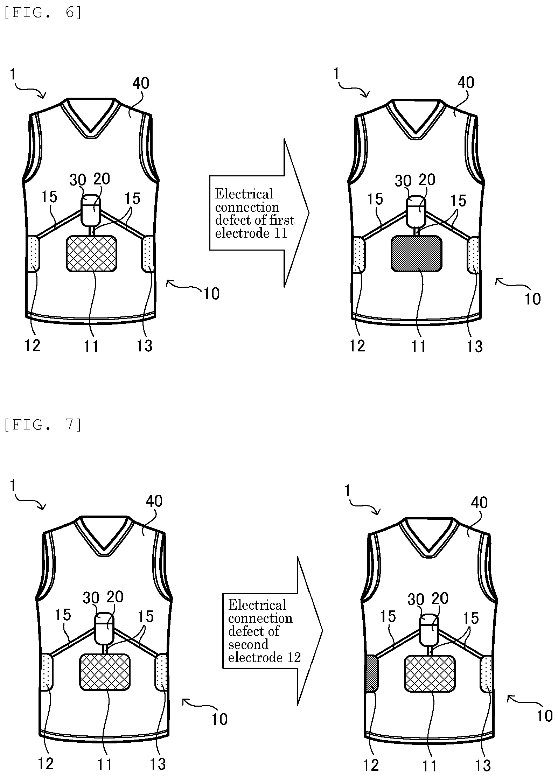

Additionally, if, for example, peel-off occurs in the single electrode and if an electrical connection defect is caused in the electrode group 10, the operation is performed as follows. For example, when the electrical connection of the electrode of the first polarity is defective, unintentional stimulation does not occur because the other electrodes of the second polarity are the same in waveform. For example, if the electrical connection of the first electrode 11 is cut when the first electrode 11 is set as the first polarity, and the second electrode 12 and the second electrode 13 are each set as the second polarity as shown in FIG. 6, stimulation does not occur in the second electrode 12 and the second electrode 13 because of the same signal. In FIG. 6, the electrode of the first polarity is shown with hatching, and the electrode of the second polarity is shown with dots, and the electrode that is defective in electrical connection is shown in gray.

On the other hand, if an electrical connection is defective in one electrode that has been set as the second polarity, the number of other electrodes of the second polarity is one or more, and the relationship of one or more electrodes of the second polarity to one electrode of the first polarity is formed, and therefore unintentional strong stimulation does not occur, and the stimulation becomes weak. For example, if the electrical connection of the second electrode 12 is defective when the first electrode 11 is set as the first polarity and if the second electrode 12 and the second electrode 13 are each set as the second polarity as shown in FIG. 7, the relationship of one electrode of the second polarity to one electrode of the first polarity is formed although the relationship of two electrodes of the second polarity to one electrode of the first polarity is originally formed, and therefore strong stimulation does not occur. In FIG. 7, the electrode of the first polarity is shown with hatching, and the electrode of the second polarity is shown with dots, and the electrode having an electrical connection defect is shown in gray.

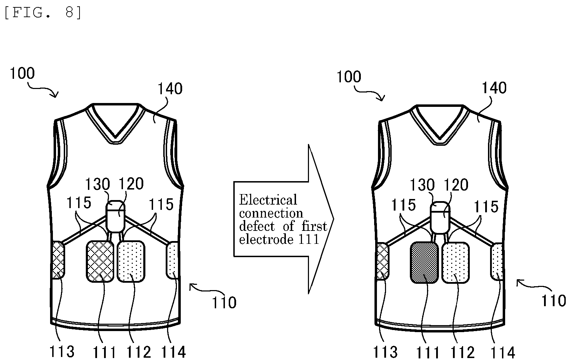

On the other hand, if, for example, peel-off occurs in one electrode so that an electrical connection becomes defective when energy is given between a plurality of electrodes and a plurality of electrodes, the operation is performed as follows. For example, an electrical stimulator 100 shown in FIG. 8 has an electrode group 110 having four electrodes, **i.e.,** having a first electrode 111 disposed correspondingly to right-hand rectus abdominis muscles, a second electrode 112 disposed correspondingly to left-hand rectus abdominis muscles, a third electrode 113 disposed correspondingly to right-hand oblique abdominal muscles, and a fourth electrode 114 disposed correspondingly to left-hand oblique abdominal muscles, and the electrical stimulator 100 allows a driving means 120 to give energy to the electrode group 110 under the condition that the first electrode 111 and the third electrode 113 are each set as the first polarity, and the second electrode 112 and the fourth electrode 114 are each set as the second polarity. Except for this, the electrical stimulator 100 is configured in the same way as the electrical stimulator 1 according to the present embodiment, and therefore, in FIG. 8, a component corresponding to each component of the electrical stimulator 1 is denoted by a reference numeral formed by adding 100 to the reference numeral of each component of the electrical stimulator 1.

If an electrical connection to the first electrode 111 is cut in the electrical stimulator 100, the relationship of the plurality of electrodes each of which has the second polarity to the single electrode having the first polarity is formed although the relationship of the plurality of electrodes each of which has the second polarity to the plurality of electrodes each of which has the first polarity is originally formed, and therefore stimulation concentrates at the third electrode 113, and unintentional strong stimulation occurs in the third electrode 113. In FIG. 8, the electrode of the first polarity is shown with hatching, and the electrode of the second polarity is shown with dots, and the electrode having an electrical connection defect is shown in gray.

As thus described, in the electrical stimulator 1 according to the present embodiment, the occurrence of unintentional strong stimulation is restrained even if an electrical connection is cut in part of the electrodes.

If an electrical connection becomes defective because of, for example, disconnection or peel-off in each electrode, this defect is detected by, for example, the connection-defect detection means 30. For example, if an electrical connection defect occurs in the first electrode 11 of the first polarity because of peel-off or the like when energy is given to the electrode group 10 under the condition that the first electrode 11 is set as the first polarity and that the other electrodes, i.e., the second and third electrodes 12 and 13 are each set as the second polarity as shown in FIG. 5, an electric current does not flow between the first electrode 11 and the second and third electrodes 12, 13, and electrical stimulation is not applied to the user. Therefore, electric potential is not applied to the base of the NPN bipolar transistor 31, and a High state detection signal is obtained from the collector.

On the other hand, if no electrical connection defect occurs in each electrode, an electric current flows between the first electrode 11 and the second and third electrodes 12, 13 as shown in, for example, FIG. 4, and electrical stimulation is applied to the user. Therefore, electric potential is applied to the base of the NPN bipolar transistor 31, and a Low state detection signal is obtained from the collector. Therefore, whether electrical stimulation has been applied to the user when energy is given to the electrode group 10 is detected by use of a difference in the state signal of the collector, and, if electrical stimulation is not applied to the user, it is detected that the electrode that has been set as the first polarity is defective in electrical connection. When the connection-defect detection means 30 detects the electrical connection defect, for example, a defect signal is output to the control means 50, and the control means 50 displays an electrode whose electrical connection is defective.

If an electrical connection defect has occurred in the electrode of the second polarity, the electrical connection defect is not detected when the electrode is set as the second polarity, and yet switching is performed among the electrodes each of which is set as the first polarity in predetermined order in the driving means 20, and therefore an electrical connection defect is detected when the electrodes are each set as the first polarity.

As thus described, according to the present embodiment, one electrode of the electrode group 10 is set as the first polarity, and the remaining plurality of electrodes are each set as the second polarity, hence making it possible to allow an electric current to flow between the single electrode of the first polarity and the remaining plurality of electrodes of the second polarity, to allow stimulation to concentrate at the single electrode of the first polarity, and to reduce stimulation in the plurality of electrodes of the second polarity. Depending on circumstances, it is possible to eliminate the stimulation of the electrode of the second polarity or to greatly weaken the stimulation thereof. Therefore, it is possible to restrain the application of unintentional strong stimulation to the other electrodes of the second polarity, for example, even if wiring disconnection has occurred or even if a contact state with the body becomes worse because of peel-off or the like in any one of the plurality of electrodes of the second polarity. Therefore, it is further possible to restrain the user from receiving an uncomfortable sensation while feeling strong stimulation or to restrain the user from being surprised at strong stimulation and from reflexively moving the user's body, and it is possible to prevent low temperature burn that is caused by the concentration of an electric current.

This effect is effective particularly when high-frequency electrical stimulation is applied. The high frequency means the range of, for example, 1 kHz to 100 kHz.

Additionally, stimulation is concentrated at one electrode of the first polarity by giving energy under the condition that the number of first polarities is one and that the number of second polarities is two or more, and switching is performed among the electrodes of the first polarity in predetermined order in the electrode group 10, and therefore it is possible to apply less-variable strong stimulation in turn in all electrodes. Additionally, it is possible to concentrate stimulation at the single electrode of the first polarity, and therefore it is possible to apply strong stimulation with lower energy than in the past.

Additionally, it is possible to easily detect defects while applying electrical stimulation if an electrical connection defect is detected by detecting whether electrical stimulation has been applied to the user when energy is given to the electrode group 10. Therefore, when peel-off of the electrode or a contact defect has occurred, it is possible to quickly correct such failures during use.

Still additionally, energy is given to the electrode group 10 under the condition that one electrode of the electrode group 10 is set as the first polarity and that the remaining plurality of electrodes are each set as the second polarity, and therefore it is possible to easily detect an electrical connection defect by use of the fact that electrical stimulation is not applied to the user if there is an electrical connection defect in the electrode set as the first polarity.

### (Second Embodiment)

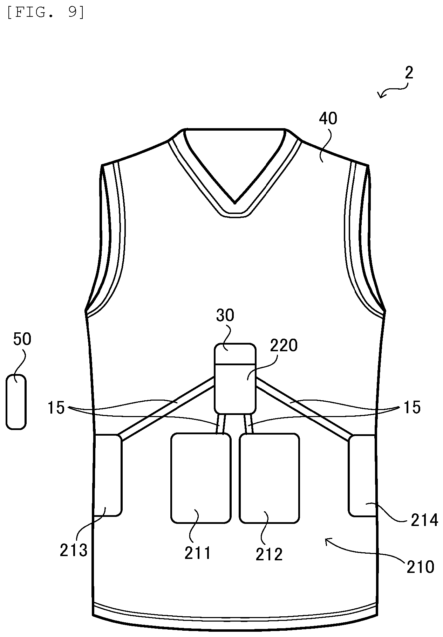

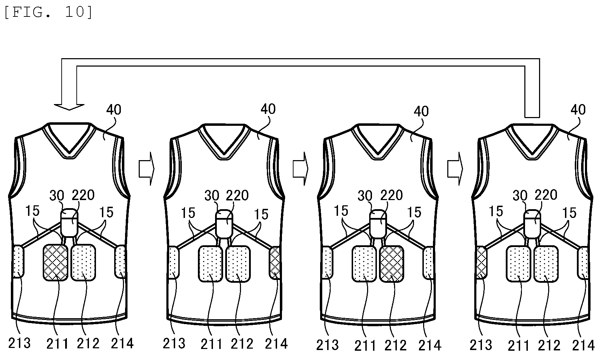

FIG. 9 represents a configuration of an electrical stimulator 2 according to a second embodiment of the present invention. FIG. 10 describes the operation of the electrical stimulator 2. The electrical stimulator 2 is configured in the same way as in the first embodiment except for the fact that an electrode group 210 and a driving means 220 differ in configuration from those of the first embodiment. Therefore, the same reference sign is given to a component identical with each component of the first embodiment, and a reference numeral formed by adding 200 is given to each corresponding component, and a detailed description of the same part is omitted.

The electrode group 210 is disposed at a trunk, such as an abdominal region, in the same way as in the first embodiment, and has at least four electrodes. In detail, the electrode group 210 has a first electrode 211 disposed correspondingly to right-hand rectus abdominis muscles, a second electrode 212 disposed correspondingly to left-hand rectus abdominis muscles, a third electrode 213 disposed correspondingly to right-hand oblique abdominal muscles, and a fourth electrode 214 disposed correspondingly to left-hand oblique abdominal muscles. Except for this, the electrode group 210 is configured in the same way as the electrode group 10 of the first embodiment.

Except for the fact that specific order in which switching among the electrodes each of which is set as the first polarity is performed differs from that of the first embodiment, the driving means 220 is configured in the same way as in the first embodiment. Preferably, the driving means 220 is configured to perform switching among the electrodes each of which is set as the first polarity in order in which the first electrode 211 and the second electrode 212 do not consecutively become the first polarity. The first electrode 211 and the second electrode 212 that are disposed correspondingly to rectus abdominis muscles are physically close to each other, and therefore, if the first and second electrodes 211 and 212 consecutively become the first polarity, muscles near the first and second electrodes 211 and 212 will consecutively receive electrical stimulation at short intervals of time, and a relaxation operation will become insufficient, and therefore early muscle fatigue will occur, or the user will receive an uncomfortable sensation. On the other hand, the third electrode 213 and the fourth electrode 214 that are disposed correspondingly to oblique abdominal muscles have a positional relationship in which the third and fourth electrodes 213 and 214 are physically distant from each other, and the third and fourth electrodes 213 and 214 are distant from the first and second electrodes 211 and 212, and therefore the aforementioned problems do not arise.

Preferably, for example, the driving means 220 is configured to repeatedly perform switching among the electrodes each of which is set as the first polarity in order of the first electrode 211, the fourth electrode 214, the second electrode 212, and the third electrode 213 as shown in FIG. 10. In FIG. 10, the electrode that is set as the first polarity is shown with hatching, and the electrode that is set as the second polarity is shown with dots.

According to the present embodiment, it is possible to obtain the same operation/effect as in the first embodiment. Particularly, if an electrical connection defect occurs in the single electrode of the second polarity, two other electrodes of the second polarity remain, and an electric current is dispersed, and therefore the occurrence of strong stimulation is restrained.

Additionally, according to the present embodiment, at least, if the first electrode 211 is disposed correspondingly to right rectus abdominis muscles, the second electrode 212 is disposed correspondingly to left rectus abdominis muscles, the third electrode 213 is disposed correspondingly to right-hand oblique abdominal muscles, and the fourth electrode 214 is disposed correspondingly to left-hand oblique abdominal muscles, and if switching among the electrodes each of which is set as the first polarity is performed in turn so that the first electrode 211 and the second electrode 212 do not consecutively become the first polarity, it is possible to restrain electrical stimulation from being consecutively applied to muscles near the first and second electrodes 211 and 212 that are disposed close to each other and to restrain a relaxation operation from becoming insufficient. Therefore, it is possible to more effectively perform a relaxation operation in the whole of the range in which the electrode group 210 is disposed, and it is possible to restrain early muscle fatigue from being caused or to restrain the user from receiving an uncomfortable sensation.

### (Third Embodiment)

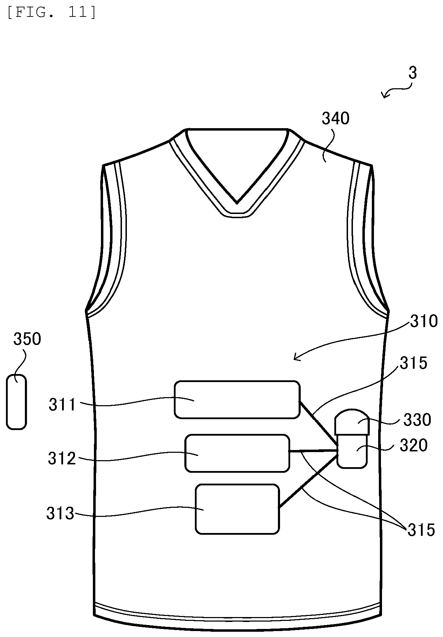

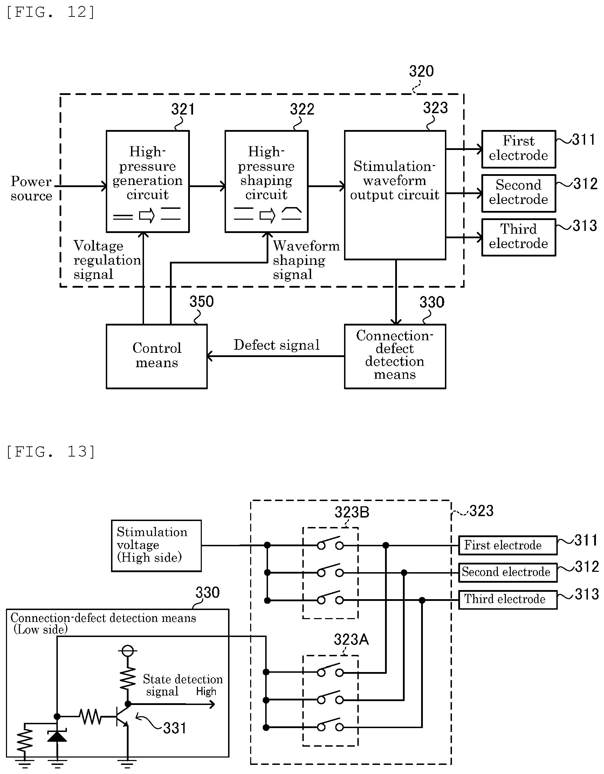

FIG. 11 represents a configuration of an electrical stimulator 3 according to a third embodiment of the present invention. FIG. 12 to FIG. 15 each represent a circuit configuration of the electrical stimulator 3. The electrical stimulator 3 applies electrical stimulation, for example, to a user, and includes an electrode group 310 having three or more electrodes, a driving means 320 that gives energy to the electrode group 310, a connection-defect detection means 330 that detects electrical connection defects, a garment 340 on which the electrode group 310 is disposed at a predetermined position, and a control means 350 that controls the driving means 320.

The electrode group 310 is, for example, brought into contact with a user's body and is disposed at a predetermined position. In the present embodiment, for example, the electrode group 310 is disposed at a trunk, such as an abdominal region, and has at least three electrodes. In detail, the electrode group 310 has a first electrode 311, a second electrode 312, and a third electrode 313 that are disposed in this order downwardly from the upper one correspondingly to rectus abdominis muscles.

Under the condition that one or more electrodes of the electrode group 310 are each set as the first polarity and that one or more other electrodes are each set as the second polarity, the driving means 320 gives energy to the electrode group 310 while performing switching among at least either the electrodes set as the first polarity or the electrodes set as the second polarity. In detail, the driving means 320 is configured to give energy to the electrode group 310, for example, under the condition that one electrode of the electrode group 310 is set as the first polarity and that one other electrode is set as the second polarity, while performing switching among at least either the electrodes each of which is set as the first polarity or the electrodes each of which is set as the second polarity in turn. This makes it possible to reduce the number of switching operations among the electrodes that are each set as the first polarity or as the second polarity and to give energy to the whole of the electrode group 310. The first polarity and the second polarity differ from each other in polarity, and, for example, the first polarity is a positive electrode whereas the second polarity is a negative electrode, or, the first polarity is a negative electrode whereas the second polarity is a positive electrode. With respect to order in which switching is performed among the electrodes that are each set as the first polarity or as the second polarity, the stimulation order can be ambulatory changed to disperse fatigue caused by electrical stimulation. It becomes possible to apply stimulation to a large range, for example, by a stimulation method for simultaneously performing switching both among the electrodes that are each set as the first polarity and among the electrodes that are each set as the second polarity, and it is also possible to disperse a period of time during which electrical stimulation is applied to muscles so as to disperse muscle fatigue. Additionally, it is also possible to apply stimulation minimally, for example, while performing switching either among the electrodes that are each set as the first polarity or among the electrodes that are each set as the second polarity.

The driving means 320 is, for example, arranged on the garment 340, and is connected to the electrode group 310, to the connection-defect detection means 330, and to the control means 350. With respect to a circuit configuration of the driving means 320, the driving means 320 has, for example, a high-pressure generation circuit 321 that is connected to a power source (not shown) and that generates a high-pressure DC voltage, a high-pressure shaping circuit 322 that is connected to the high-pressure generation circuit 321 and that shapes a waveform, and a stimulation-waveform output circuit 323 that is connected to the high-pressure shaping circuit 322 and to each electrode of the electrode group 310 and that outputs a stimulation waveform to each electrode of the electrode group 310.

Preferably, the high-pressure generation circuit 321 is configured to be capable of changing a to-be-generated voltage value by the control means 350 although the to-be-generated voltage value may be fixed at a constant value. Preferably, the high-pressure shaping circuit 322 is configured to be capable of changing a waveform by the control means 350 although the waveform that has been shaped may be fixed in a constant waveform. The stimulation-waveform output circuit 323 is configured to apply a voltage between an electrode and an electrode, for example, under the condition that one electrode of the electrode group 310 is set as the first polarity and that one other electrode is set as the second polarity, while performing switching among at least either the electrodes each of which is set as the first polarity or the electrodes each of which is set as the second polarity in turn.

The connection-defect detection means 330 detects electrical connection defects by detecting whether electrical stimulation has been applied to a user when energy is given to the electrode group 310. For example, wiring breakage, peel-off from a user's body, and contact failure in each electrode of the electrode group 310 can be mentioned as the electrical connection defects.

A configuration shown in, for example, FIG. 13 can be mentioned as the circuit configuration of the connection-defect detection means 330. For example, the connection-defect detection means 330 has a grounded-emitter NPN bipolar transistor 331, and a Low-side switch group 323A corresponding to each electrode of the stimulation-waveform output circuit 323 is connected to a base of the NPN bipolar transistor 331. Hence, in the connection-defect detection means 330, the Low-side switch group 323A and a High-side switch group 323B corresponding to each electrode of the stimulation-waveform output circuit 323 are all brought into an open state as shown in, for example, FIG. 13, and, when energy is not given to the electrode group 310, a collector of the NPN bipolar transistor 331 becomes High, and, as a result, it is possible to obtain a High state detection signal from the collector.

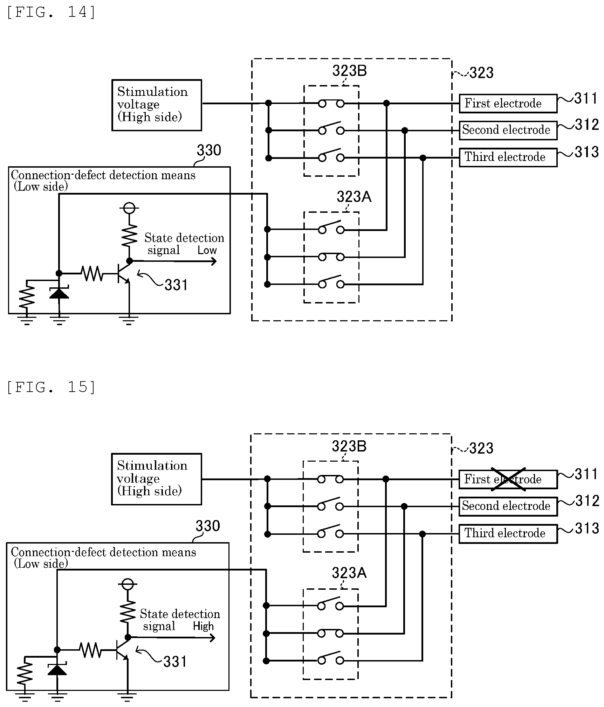

Additionally, for example, as shown in FIG. 14, switches corresponding to the first electrode 311 are brought into a closed state, whereas the other switches are brought into an open state in the High-side switch group 323B of the stimulation-waveform output circuit 323, and switches corresponding to the second electrode 312 are brought into a closed state, whereas the other switches are brought into an open state in the Low-side switch group 323A, and, when energy is given to the electrode group 310 under the condition that the first electrode 311 is set as the first polarity, and the second electrode 312 is set as the second polarity, an electric current flows through a user's body between the first electrode 311 and the second electrode 312, and electric potential is applied to the base of the NPN bipolar transistor 331, and a collector current flows, and the collector becomes Low, and, as a result, it is possible to obtain a Low state detection signal from the collector. In other words, the state detection signal of the collector becomes a signal that differs in signal level from the signal obtained when energy is not given to the electrode group 310.

However, even when energy is given to the electrode group 310 under the condition that the first electrode 311 is set as the first polarity and that the second electrode 312 is set as the second polarity in the same way as in FIG. 14, an electric current does not flow between the first electrode 311 and the second electrode 312, and electrical stimulation is not applied to the user as shown in FIG. 15 if an electrical connection defect occurs in the first electrode 311 because of peel-off or the like, and therefore electric potential is not applied to the base of the NPN bipolar transistor 331, and the collector becomes High, and a High state detection signal is obtained from the collector. In other words, the state detection signal of the collector becomes the same signal as when energy is not given to the electrode group 310. Therefore, the state signal of the collector depends on whether electrical stimulation has been applied to the user when energy is given to the electrode group 310.

Thus, the connection-defect detection means 330 is configured to detect whether electrical stimulation has been applied to the user when energy is given to the electrode group 310 by detecting the state detection signal of the collector of the NPN bipolar transistor 331 so as to detect an electrical connection defect. In the above description, a case has been described in which a switch corresponding to the electrode set as the first polarity is brought into a closed state in the High-side switch group 323B of the stimulation-waveform output circuit 323, whereas a switch corresponding to the electrode set as the first polarity is brought into an open state in the Low-side switch group 323A, and yet the same applies to a case in which a switch corresponding to the electrode set as the first polarity is brought into a closed state in the Low-side switch group 323A, whereas a switch corresponding to the electrode set as the first polarity is brought into an open state in the High-side switch group 323B.

The connection-defect detection means 330 is, for example, arranged on the garment 340, and is connected to the driving means 320 and to the control means 350. Preferably, the connection-defect detection means 330 is configured, for example, to output a defect signal to the control means 350 when an electrical connection defect is detected.

Any type of clothing item may be used as the garment 340 as long as it is worn on a user's body. For example, the garment 340 may be a closing item such as a running shirt or a T-shirt as shown in FIG. 11 with which the trunk is covered, and may be a strip-shaped or belt-shaped item that is worn around a user's abdominal region. Although a sleeveless clothing item is shown in FIG. 11, a sleeved clothing item may also be used, and may have a part with which not only an upper half of the body but also a lower half of the body is covered. Additionally, preferably, the garment 340 is made of a stretchy material. The reason is that such a stretchy material enables the electrode group 310 to fit close to the body.

Preferably, the control means 350 is configured to detect an electrode having an electrical connection defect from a relationship between an electrode set as the first polarity or as the second polarity when an electrical connection is defective and an electrode set as the first polarity or as the second polarity when an electrical connection is normal, and to display that defective electrode. For example, the control means 350 is configured such that if an electrical connection becomes defective when energy is given under the condition that one electrode of the electrode group 310 is set as the first polarity and that one other electrode is set as the second polarity and if an electrical connection becomes normal when the electrode set as the first polarity is switched, an electrical connection is detected being defective in the electrode set as the first polarity before such switching, and, if an electrical connection becomes normal when the electrode set as the second polarity is switched, an electrical connection is detected being defective in the electrode set as the second polarity before such switching.

The control means 350 is, for example, configured to be capable of performing control while being kept on hand without being arranged on the garment 340. The connection between the control means 350 and the driving means 320 and connection-defect detection means 330 may be wired or may be wireless. Additionally, preferably, the control means 350 is provided with, for example, a switch that issues a drive command and a stop command to the driving means 320.

Each electrode of the electrode group 310 can be made of, for example, a conductor in which an electroconductive polymer adheres to a base. Any kind of materials of which the base is made may be used, and it is preferable to use fiber, for example, that includes at least one kind of natural fiber, such as silk or cotton, and artificial fiber, such as synthetic fiber. According to the invention, poly-3,4-ethylenedioxythiophene (PEDOT) is used as the electroconductive polymer. The conductor can be obtained, for example, by polymerizing monomers of the electroconductive polymer adhering to the base by use of an oxidizer. In that case, either a dopant that allows the electroconductive polymer to exhibit electroconductivity or a thickening agent may be allowed to adhere to the base together with the monomers of the electroconductive polymer.

Preferably, for example, iron salt can be mentioned as the oxidizer. Preferably, for example, p-toluenesulfonic acid can be mentioned as the dopant, and it is more preferable to use iron salt of p-toluenesulfonic acid (pTS) because that can be allowed to function as the oxidizer and as the dopant. Besides, acetonitrile, trifluoroacetic acid, etc., can be mentioned as the dopant. The thickening agent is used to reduce the bleeding of an electroconductive polymer and to accelerate the polymerization reaction of monomers. Preferably, an agent that does not react to the polymerization reaction of the electroconductive polymer is used as the thickening agent, and, preferably, glycerol, polyethylene glycol, gelatin, or polysaccharides can be mentioned as examples of the thickening agent.

Each electrode of the electrode group 310 may be, for example, formed directly on the garment 3 by using fiber, of which the garment 340 is made, as the base and by allowing an electroconductive polymer to adhere thereto by printing or the like, and may be arranged by bonding or sewing a conductor, which is formed by allowing an electroconductive polymer to adhere to a base prepared independently of the garment 340, onto the garment 340. Lead wires 315 by which each electrode of the electrode group 310 and the driving means 320 are connected together can also be formed in the same way as the electrode group 310.

This electrical stimulator 3 is used as follows. First, a user wears the garment 340 on a user's body to dispose each electrode of the electrode group 310 at a predetermined position of the body. Thereafter, the control means 350 issues a drive command to the driving means 320. In the driving means 320, for example, one electrode of the electrode group 310 is set as the first polarity, and one other electrode is set as the second polarity, and energy is given to the electrode group 310 while performing switching among at least either the electrodes each of which is set as the first polarity or the electrodes each of which is set as the second polarity in turn. Hence, an electric current flows between the single electrode of the first polarity and the other single electrode of the second polarity.

For example, if energy is given to the electrode group 310 under the condition that the first electrode 311 is set as the first polarity and that the second electrode 312 is set as the second polarity as shown in FIG. 14, an electric current flows between the first electrode 311 and the second electrode 312, and electrical stimulation is applied to the user. Hence, electric potential is applied to the base of the NPN bipolar transistor 331, and a Low state detection signal is obtained from the collector. On the other hand, for example, if an electrical connection defect occurs in the first electrode 11 of the first polarity because of peel-off or the like, an electric current does not flow between the first electrode 311 and the second electrode 312, and electrical stimulation is not applied to the user as shown in FIG. 15. Therefore, electric potential is not applied to the base of the NPN bipolar transistor 331, and a High state detection signal is obtained from the collector.

In the connection-defect detection means 330, whether electrical stimulation has been applied to the user when energy is given to the electrode group 310 is detected by use of a difference in the state signal of the collector, and, when an electrical connection defect is detected, the connection-defect detection means 330 outputs a defect signal, for example, to the control means 350. In the control means 350, an electrode having an electrical connection defect is detected from a relationship between an electrode set as the first polarity or as the second polarity when an electrical connection is defective and an electrode set as the first polarity or as the second polarity when an electrical connection is normal. For example, if an electrical connection becomes defective when the first electrode 311 is set as the first polarity and when the second electrode 312 is set as the second polarity and if electrical connection becomes normal when the electrode that is set as the first polarity is switched to the third electrode 313, the first electrode 311 is detected having an electrical connection defect. Additionally, for example, if an electrical connection becomes defective when the first electrode 311 is set as the first polarity and when the second electrode 312 is set as the second polarity and if an electrical connection becomes normal when the electrode of the second polarity is switched to the third electrode 313, the second electrode 312 is detected having an electrical connection defect. The control means 350 displays the electrode having an electrical connection defect detected here.

As thus described, according to the present embodiment, energy is given to the electrode group 310 while performing switching among the electrodes, which are each set as the first polarity and as the second polarity, of the electrode group 310 having three or more electrodes, and an electrical connection defect is detected by detecting whether electrical stimulation has been applied to the user when energy is given to the electrode group 310, and therefore it is possible to easily detect a defect while applying electrical stimulation. Therefore, when peel-off of the electrode or a contact defect has occurred, it is possible to quickly correct such failures during use.

### (Fourth Embodiment)

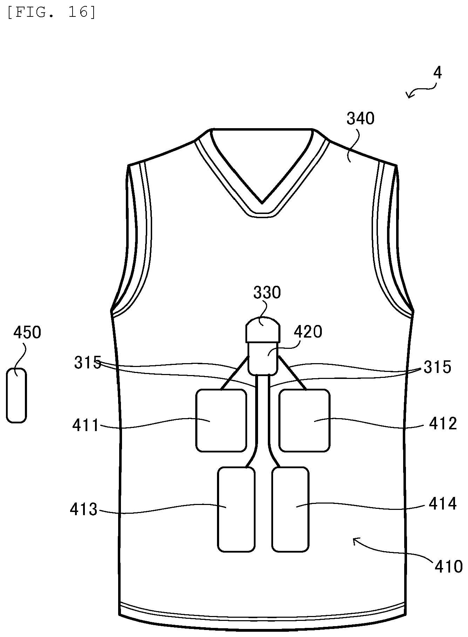

FIG. 16 represents a configuration of an electrical stimulator 4 according to a fourth embodiment of the present invention. The electrical stimulator 4 is configured in the same way as in the third embodiment except for the fact that an electrode group 410, a driving means 420, and a control means 450 differ in configuration from those of the third embodiment. Therefore, the same reference sign is given to a component identical with each component of the third embodiment, and a reference numeral formed by changing the hundreds place digit to 4 is given to each corresponding component, and a detailed description of the same part is omitted.

The electrode group 410 has, for example, four or more electrodes that are disposed so as to come into contact with a trunk, such as an abdominal region. In detail, for example, the electrode group 410 has a first electrode 411 disposed correspondingly to upper right-hand rectus abdominis muscles, a second electrode 412 disposed correspondingly to upper left-hand rectus abdominis muscles, a third electrode 413 disposed correspondingly to lower right-hand rectus abdominis muscles, and a fourth electrode 414 disposed correspondingly to lower left-hand rectus abdominis muscles. Except for this, the electrode group 410 is configured in the same way as the electrode group 310 of the third embodiment.

The driving means 420 is configured in the same way as in the third embodiment except for the fact that energy is given to the electrode group 410 while selectively performing switching among the electrodes set as the first polarity and as the second polarity in accordance with a muscle action pattern under the condition that one or more electrodes of the electrode group 410 are each set as the first polarity and that one or more other electrodes are each set as the second polarity. Preferably, for example, the driving means 420 is configured to selectively perform switching among the electrodes set as the first polarity and as the second polarity in accordance with a muscle action pattern under the condition that one or more electrodes among the first electrode 411, the second electrode 412, the third electrode 413, and the fourth electrode 414 are each set as the first polarity and that the remaining one or more electrodes are each set as the second polarity.

In detail, for example, an electrode (not shown) for measuring an electromyogram (EMG) signal is attached, and an EMG signal produced when a muscle contraction occurs is measured, and signal processing is applied to this EMG signal, and, as a result, a muscle contraction pattern and muscle-strength information are calculated, and the muscle contraction pattern obtained here is input into a neural net, and a muscle action is subjected to machine learning, and the posterior probability of the muscle action is calculated from a muscle contraction state in consideration of time-series characteristics. A posterior probability calculated by the neural net and energy by which a preformed action is performed are calculated by use of a posterior probability based on a dynamic action model, and, with respect to an action pattern according to which muscles act, a joint angle formed when previous-stimulation energy is given is measured, and an electrical stimulation pattern that causes a joint motion is beforehand constructed, and it is possible to apply the stimulation of an electrical stimulation pattern according to which muscles act.

In an electrical stimulation pattern construction method, an EMG signal is converted into a digital signal in an analogue-to-digital conversion, and is subjected to full-wave rectification, and is then subjected to a smoothing process by a secondary low-pass filter in signal processing. The EMG signal observed during rest is eliminated, and a signal is obtained by normalizing a maximum value of each channel. From muscle-strength information, whether action transmission has been made is determined by a threshold value. The neural net that performs machine learning is allowed to learn an electromyogram pattern that has been measured during each action, and then the electromyogram pattern is constructed for muscle actions. Except for this, the driving means 420 is configured in the same way as the driving means 320 of the third embodiment.

Preferably, the control means 450 is configured to detect an electrode having an electrical connection defect from a relationship between an electrode set as the first polarity or as the second polarity when an electrical connection is defective and an electrode set as the first polarity or as the second polarity when an electrical connection is normal, and to display that defective electrode. For example, the control means 450 is configured such that if an electrical connection becomes defective when energy is given under the condition that one electrode of the electrode group 410 is set as the first polarity and that one other electrode is set as the second polarity and if an electrical connection becomes normal when switching is performed among the electrodes each of which is set as the first polarity or when the number of the electrodes each of which is set as the first polarity is increased, an electrical connection is detected being defective in the electrode set as the first polarity before such switching, and if an electrical connection becomes normal when switching is performed among the electrodes each of which is set as the second polarity or when the number of the electrodes each of which is set as the second polarity is increased, an electrical connection is detected being defective in the electrode set as the second polarity before such switching.

Additionally, for example, the control means 450 is configured to detect that the electrode that has been set as the first polarity has an electrical connection defect if electrical connection becomes defective when energy is given under the condition that one electrode of the electrode group 410 is set as the first polarity and that the other plurality of electrodes are each set as the second polarity, and is configured to detect that the electrode that has been set as the second polarity has an electrical connection defect if an electrical connection becomes defective when energy is given under the condition that one electrode of the electrode group 410 is set as the second polarity and that the other plurality of electrodes are each set as the first polarity.

Still additionally, for example, the control means 450 is configured such that if an electrical connection becomes defective when energy is given under the condition that plurality of electrodes of the electrode group 410 are each set as the first polarity and that the other plurality of electrodes are each set as the second polarity and if an electrical connection is normal when switching is performed among the electrodes that are each set as the first polarity, an electrical connection is detected being defective in the electrodes each of which has been set as the first polarity before such switching, and if an electrical connection is normal when switching is performed among the electrodes that are each set as the second polarity, an electrical connection is detected being defective in the electrodes each of which has been set as the second polarity before such switching. Except for this, the control means 450 is configured in the same way as the control means 350 of the third embodiment.

In this electrical stimulator 4, for example, a user wears the garment 340 on a user's body to dispose each electrode of the electrode group 410 at a predetermined position of the body, and the control means 450 issues a drive command to the driving means 420, and then energy is given to the electrode group 410 while selectively performing switching among the electrodes set as the first polarity and as the second polarity in accordance with a muscle action pattern under the condition that one or more electrodes of the electrode group 410 are each set as the first polarity and that one or more other electrodes are each set as the second polarity. At that time, in the connection-defect detection means 330, whether electrical stimulation has been applied to the user when energy is given to the electrode group 410 is detected by use of a difference in the state signal of the collector, and, when an electrical connection defect is detected, the connection-defect detection means 330 outputs a defect signal, for example, to the control means 450 in the same way as in the third embodiment. In the control means 450, an electrode having an electrical connection defect is detected from a relationship between an electrode set as the first polarity or as the second polarity when an electrical connection is defective and an electrode set as the first polarity or as the second polarity when an electrical connection is normal, and the electrode having an electrical connection defect detected here is displayed.

As thus described, according to the present embodiment, energy is given to the electrode group 410 while performing switching among the electrodes, which are each set as the first polarity and as the second polarity, of the electrode group 410 having three or more electrodes, and an electrical connection defect is detected by detecting whether electrical stimulation has been applied to the user when energy is given to the electrode group 410, and therefore it is possible to easily detect a defect while applying electrical stimulation. Therefore, when peel-off of the electrode or a contact defect has occurred, it is possible to quickly correct such failures during use.

### (Fifth Embodiment)

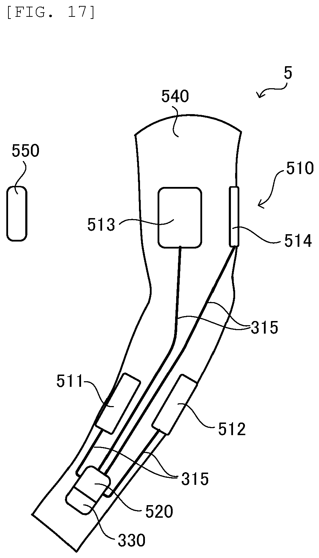

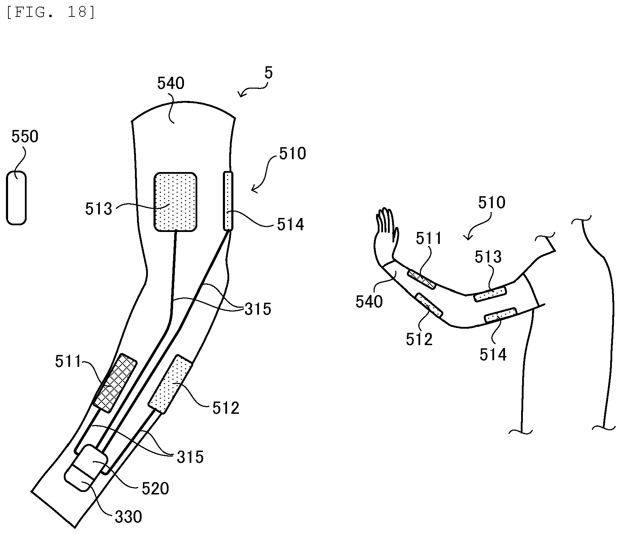

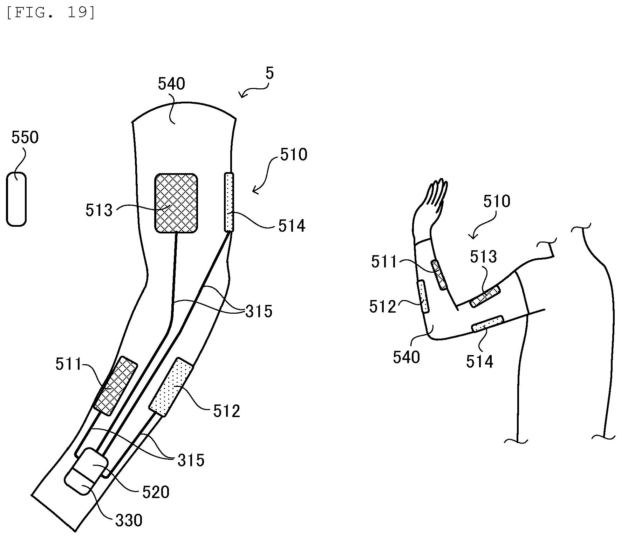

FIG. 17 represents a configuration of an electrical stimulator 5 according to a fifth embodiment of the present invention. FIG. 18 and FIG. 19 are each a view to describe the operation of the electrical stimulator 5. The electrical stimulator 5 is configured in the same way as in the third embodiment except for the fact that an electrode group 510, a driving means 520, a garment 540, and a control means 550 differ in configuration from those of the third embodiment. Therefore, the same reference sign is given to a component identical with each component of the third embodiment, and a reference numeral formed by changing the hundreds place digit to 5 is given to each corresponding component, and a detailed description of the same part is omitted.

The electrode group 510 has, for example, three or more electrodes that are disposed so as to come into contact with a right arm or a left arm. FIG. 17 to FIG. 19 each show a case of the left arm. In detail, preferably, the electrode group 510 has a first electrode 511 disposed correspondingly to an antebrachial musculus extensor carpi radialis brevis, a second electrode 512 disposed correspondingly to an antebrachial musculus flexor carpi radialis, a third electrode 513 disposed correspondingly to a biceps brachii muscle, and a fourth electrode 514 disposed correspondingly to a triceps brachii muscle. Except for this, the electrode group 510 is configured in the same way as the electrode group 310 of the third embodiment.

The driving means 520 is configured in the same way as in the third embodiment except for the fact that energy is given to the electrode group 510 while selectively performing switching among the electrodes set as the first polarity and as the second polarity in accordance with a muscle action pattern under the condition that one or more electrodes of the electrode group 510 are each set as the first polarity and that one or more other electrodes are each set as the second polarity. Preferably, for example, the driving means 520 is configured to selectively perform switching among the electrodes set as the first polarity and as the second polarity in accordance with a muscle action pattern under the condition that one or more electrodes among the first electrode 511, the second electrode 512, the third electrode 513, and the fourth electrode 514 are each set as the first polarity and that the remaining one or more electrodes are each set as the second polarity.

In detail, preferably, the first electrode 511 is set as the first polarity, and the remaining other electrodes (i.e., the second electrode 512, the third electrode 513, and the fourth electrode 514) are each set as the second polarity, for example, when the user slightly bends his/her forearm as shown in FIG. 18, whereas the first electrode 511 and the third electrode 513 are each set as the first polarity, and the remaining other electrodes (i.e., the second electrode 512 and the fourth electrode 514) are each set as the second polarity, for example, when the user greatly bends his/her forearm as shown in FIG. 19. In FIG. 18 and FIG. 19, the electrode set as the first polarity is shown with hatching, and the electrode set as the second polarity is shown with dots. This makes it possible to effectively assist or burden the forearm-bending motion by stimulating the muscles. Except for this, the driving means 520 is configured in the same way as the driving means 320 of the third embodiment.

For example, a sleeved clothing item or an arm cover can be mentioned as the garment 540. FIG. 17 to FIG. 19 each show an example of a left arm cover. Except for this, the garment 540 is configured in the same way as the garment 340 of the third embodiment. Preferably, the control means 550 is configured to detect an electrode having an electrical connection defect from a relationship between an electrode set as the first polarity or as the second polarity when an electrical connection is defective and an electrode set as the first polarity or as the second polarity when an electrical connection is normal, and to display that defective electrode. In detail, preferably, the control means 550 is configured to detect an electrode having an electrical connection defect in the same way as the control means 450 of the fourth embodiment. Except for this, the control means 550 is configured in the same way as the control means 350 of the third embodiment.

In this electrical stimulator 5, for example, a user wears the garment 540 on a user's body to dispose each electrode of the electrode group 510 at a predetermined position of the body, and the control means 550 issues a drive command to the driving means 520, and then energy is given to the electrode group 510 while selectively performing switching among the electrodes set as the first polarity and as the second polarity in accordance with a muscle action pattern under the condition that one or more electrodes of the electrode group 510 are each set as the first polarity and that one or more other electrodes are each set as the second polarity. At that time, in the connection-defect detection means 330, whether electrical stimulation has been applied to the user when energy is given to the electrode group 510 is detected by use of a difference in the state signal of the collector, and, when an electrical connection defect is detected, the connection-defect detection means 330 outputs a defect signal, for example, to the control means 550 in the same way as in the third embodiment. In the control means 550, an electrode having an electrical connection defect is detected from a relationship between an electrode set as the first polarity or as the second polarity when an electrical connection is defective and an electrode set as the first polarity or as the second polarity when an electrical connection is normal, and the electrode having an electrical connection defect detected here is displayed.

As thus described, according to the present embodiment, energy is given to the electrode group 510 while performing switching among the electrodes, which are each set as the first polarity and as the second polarity, of the electrode group 510 having three or more electrodes, and an electrical connection defect is detected by detecting whether electrical stimulation has been applied to the user when energy is given to the electrode group 510, and therefore it is possible to easily detect a defect while applying electrical stimulation. Therefore, when peel-off of the electrode or a contact defect has occurred, it is possible to quickly correct such failures during use.

### (Sixth Embodiment)

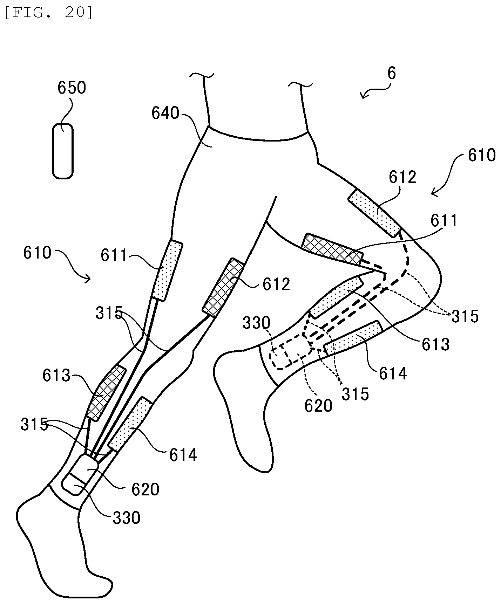

FIG. 20 represents a configuration of an electrical stimulator 6 according to a sixth embodiment of the present invention. The electrical stimulator 6 is configured in the same way as in the third embodiment except for the fact that an electrode group 610, a driving means 620, a garment 640, and a control means 650 differ in configuration from those of the third embodiment. Therefore, the same reference sign is given to a component identical with each component of the third embodiment, and a reference numeral formed by changing the hundreds place digit to 6 is given to each corresponding component, and a detailed description of the same part is omitted.

The electrode group 610 has, for example, three or more electrodes that are disposed so as to come into contact with a right leg or a left leg. In detail, preferably, the electrode group 610 has a first electrode 611 disposed correspondingly to a biceps femoris muscle, a second electrode 612 disposed correspondingly to a quadriceps femoris muscle, a third electrode 613 disposed correspondingly to a triceps surae muscle, and a fourth electrode 614 disposed correspondingly to a tibialis anterior muscle. FIG. 20 shows a case in which the first electrode 611, the second electrode 612, the third electrode 613, and the fourth electrode 614 are disposed at the right leg, and are disposed at the left leg. Except for this, the electrode group 610 is configured in the same way as the electrode group 310 of the third embodiment.

The driving means 620 is configured in the same way as in the third embodiment except for the fact that energy is given to the electrode group 610 while selectively performing switching among the electrodes set as the first polarity and as the second polarity in accordance with a muscle action pattern under the condition that one or more electrodes of the electrode group 610 are each set as the first polarity and that one or more other electrodes are each set as the second polarity. Preferably, for example, the driving means 620 is configured to selectively perform switching among the electrodes set as the first polarity and as the second polarity in accordance with a muscle action pattern under the condition that one or more electrodes among the first electrode 611, the second electrode 612, the third electrode 613, and the fourth electrode 614 are each set as the first polarity and that the remaining one or more electrodes are each set as the second polarity.

In detail, preferably, the first electrode 611 is set as the first polarity, and the remaining other electrodes (i.e., the second electrode 612, the third electrode 613, and the fourth electrode 614) are each set as the second polarity when the user raises the knee as shown in FIG. 20, whereas the second electrode 612 and the third electrode 613 are each set as the first polarity, and the remaining other electrodes (i.e., the first electrode 611 and the fourth electrode 614) are each set as the second polarity when the user stretches the leg and kicks the ground, for example. This makes it possible to effectively assist or burden the walking/running motion by stimulating the muscles. In FIG. 20, the electrode set as the first polarity is shown with hatching, and the electrode set as the second polarity is shown with dots in a case where the user raises the knee of the left leg, and stretches the leg and kicks the ground. Additionally, FIG. 20 shows a case in which the driving means 620 and the connection-defect detection means 330 for the right leg are disposed outside the right leg and in which the driving means 620 and the connection-defect detection means 330 for the left leg are disposed outside the left leg. Except for this, the driving means 620 is configured in the same way as the driving means 320 of the third embodiment.

For example, a with-crotch clothing item, such as leggings, can be mentioned as the garment 640. Except for this, the garment 640 is configured in the same way as the garment 340 of the third embodiment. Preferably, the control means 650 is configured to detect an electrode having an electrical connection defect from a relationship between an electrode set as the first polarity or as the second polarity when an electrical connection is defective and an electrode set as the first polarity or as the second polarity when an electrical connection is normal, and to display that defective electrode. In detail, preferably, the control means 650 is configured to detect an electrode having an electrical connection defect in the same way as the control means 450 of the fourth embodiment.

In this electrical stimulator 6, for example, a user wears the garment 640 on a user's body to dispose each electrode of the electrode group 610 at a predetermined position of the body, and the control means 650 issues a drive command to the driving means 620, and then energy is given to the electrode group 610 while selectively performing switching among the electrodes set as the first polarity and as the second polarity in accordance with a muscle action pattern under the condition that one or more electrodes of the electrode group 610 are each set as the first polarity and that one or more other electrodes are each set as the second polarity. At that time, in the connection-defect detection means 330, whether electrical stimulation has been applied to the user when energy is given to the electrode group 610 is detected by use of a difference in the state signal of the collector, and, when an electrical connection defect is detected, the connection-defect detection means 330 outputs a defect signal, for example, to the control means 650 in the same way as in the third embodiment. In the control means 650, an electrode having an electrical connection defect is detected from a relationship between an electrode set as the first polarity or as the second polarity when an electrical connection is defective and an electrode set as the first polarity or as the second polarity when an electrical connection is normal, and the electrode having an electrical connection defect detected here is displayed.

As thus described, according to the present embodiment, energy is given to the electrode group 610 while performing switching among the electrodes, which are each set as the first polarity and as the second polarity, of the electrode group 610 having three or more electrodes, and an electrical connection defect is detected by detecting whether electrical stimulation has been applied to the user when energy is given to the electrode group 610, and therefore it is possible to easily detect a defect while applying electrical stimulation. Therefore, when peel-off of the electrode or a contact defect has occurred, it is possible to quickly correct such failures during use.

Although the present invention has been described with reference to the embodiments as above, the present invention can be variously modified without being limited to the aforementioned embodiments. For example, although the electrode group 10 (210, 310, 410, 510, 610) is arranged on the garment 40 (340, 540, 640) as described in the aforementioned embodiments, each electrode of the electrode group 10 (210, 310, 410, 510, 610) may be individually arranged directly on the body, or electrodes of the electrode group 10 (210, 310, 410, 510, 610) may be collectively arranged on an arrangement member so as to be disposed at a predetermined position of the body, and may be collectively disposed at the predetermined position of the body by fixing the arrangement member to the body.

Additionally, although the electrode group 10 (210, 310, 410, 510, 610) has three or four electrodes as described in detail in the aforementioned embodiments, the present invention is applicable to a case in which the electrode group has at least three electrodes, and the same effect can be obtained even if the number of electrodes of the electrode group 10 (210, 310, 410, 510, 610) is five or more.

Still additionally, although the position at which the electrode is arranged has been specifically described in the aforementioned embodiments, the present invention is applicable regardless of the position at which the electrode is disposed. For example, the electrode may be disposed at another position of the abdominal region, or may be disposed on the back without being limited to the abdominal region, or may be disposed on the back and in the abdominal region. Furthermore, the electrode may be disposed at another position of the arm or of the leg.

Still additionally, although each component has been specifically described in the aforementioned embodiments, all components are not necessarily required to be included, and other components may be provided.

### Reference Signs List

1, 2, 3, 4, 5, 6 ... electrical stimulator, 10, 210, 310, 410, 510, 610 ... electrode group, 11, 211, 311, 411, 511, 611 ... first electrode, 12, 212, 312, 412, 512, 612 ... second electrode, 13, 213, 313, 413, 513, 613 ... third electrode, 214, 414, 514, 614 ... fourth electrode, 15, 315 ... lead wire, 20, 220, 320, 420, 520, 620 ... driving means, 21, 321 ... high-pressure generation circuit, 22, 322 ... high-pressure shaping circuit, 23, 323 ... stimulation-waveform output circuit, 30, 330 ... connection-defect detection means, 40, 340, 540, 640 ... garment, 50, 350, 450, 550, 650 ... control means

## Claims

1. An electrical stimulator (1, 2, 3, 4, 5, 6) that is configured to apply electrical stimulation to a user, the electrical stimulator (1, 2, 3, 4, 5, 6) comprising:
an electrode group (10, 210, 310, 410, 510, 610) that has three or more electrodes (11, 12, 13, 14, 211, 212, 213, 214, 311, 312, 313, 314, 411, 412, 413, 414, 511, 512, 513, 514, 611, 612, 613, 614) that are disposed so as to come into contact with the user;
a driving means (20, 220, 320, 420, 520, 620) that is configured to give energy to the electrode group (10, 210, 310, 410, 510, 610), under a condition that one or more electrodes of the electrode group (10, 210, 310, 410, 510, 610) are each set as a first polarity and that one or more other electrodes are each set as a second polarity, while performing switching among at least either the electrodes each of which is set as the first polarity or the electrodes each of which is set as the second polarity; and
a connection-defect detection means (30, 330) comprising a grounded-emitter NPN bipolar transistor (31, 331), wherein the connection-defect detection means (30, 330) is configured to detect an electrical connection defect by setting one of the electrodes as the first polarity while setting the others of the electrodes as the second polarity, to electrically detect whether electrical stimulation has been applied to the user when energy is given to the one of the electrodes, and to repeat the setting and detecting with another one of the electrodes being set as the first polarity,
each electrode consisting of a conductor in which a poly-3,4-ethylenedioxythiophene as an electroconductive polymer adheres to a base made of fiber.

2. The electrical stimulator (1, 2, 3, 4, 5, 6) according to claim 1, wherein the driving means (20, 220, 320, 420, 520, 620) is configured to give energy to the electrode group (10, 210, 310, 410, 510, 610), under a condition that one electrode of the electrode group (10, 210, 310, 410, 510, 610) is set as the first polarity and that remaining plurality of electrodes are each set as the second polarity, while performing switching among electrodes each of which is set as the first polarity in predetermined order.

3. The electrical stimulator (1, 2, 3, 4, 5, 6) according to claim 2, wherein, if electrical stimulation is not applied to the user when energy is given to the electrode group (10, 210, 310, 410, 510, 610), the connection-defect detection means (30, 330) is configured to detect that there is an electrical connection defect in the electrode set as the first polarity.

4. The electrical stimulator (1, 2, 3, 4, 5, 6) according to claim 2, further comprising a garment (40, 340, 540, 640) configured to dispose each electrode (11, 12, 13, 14, 211, 212, 213, 214, 311, 312, 313, 314, 411, 412, 413, 414, 511, 512, 513, 514, 611, 612, 613, 614) of the electrode group (10, 210, 310, 410, 510, 610) at a predetermined position of the body of the user, wherein at least a first electrode is disposed correspondingly to rectus abdominis muscles, a second electrode is disposed correspondingly to right-hand oblique abdominal muscles, and a third electrode is disposed correspondingly to left-hand oblique abdominal muscles.

5. The electrical stimulator according to claim 2, further comprising a garment (40, 340, 540, 640) configured to dispose each electrode (11, 12, 13, 14, 211, 212, 213, 214, 311, 312, 313, 314, 411, 412, 413, 414, 511, 512, 513, 514, 611, 612, 613, 614) of the electrode group (10, 210, 310, 410, 510, 610) at a predetermined position of the body of the user, wherein at least a first electrode is disposed correspondingly to right rectus abdominis muscles, a second electrode is disposed correspondingly to left rectus abdominis muscles, a third electrode is disposed correspondingly to right-hand oblique abdominal muscles, and a fourth electrode is disposed correspondingly to left-hand oblique abdominal muscles, and
the driving means (20, 220, 320, 420, 520, 620) is configured to perform switching among the electrodes set as the first polarity so that the first electrode and the second electrode do not consecutively become the first polarity.

6. The electrical stimulator (1, 2, 3, 4, 5, 6) according to claim 5, wherein the driving means (20, 220, 320, 420, 520, 620) is configured to perform switching among the electrodes each of which is set as the first polarity in order of the first electrode, the fourth electrode, the second electrode, and the third electrode.

7. The electrical stimulator (1, 2, 3, 4, 5, 6) according to claim 1, wherein the driving means (20, 220, 320, 420, 520, 620) is configured to give energy to the electrode group (10, 210, 310, 410, 510, 610), under a condition that one electrode of the electrode group (10, 210, 310, 410, 510, 610) is set as the first polarity and that one other electrode is set as the second polarity, while performing switching among at least either the electrodes each of which is set as the first polarity or the electrodes each of which is set as the second polarity in turn.

8. The electrical stimulator (1, 2, 3, 4, 5, 6) according to claim 1, further comprising a garment (40, 340, 540, 640) configured to dispose each electrode (11, 12, 13, 14, 211, 212, 213, 214, 311, 312, 313, 314, 411, 412, 413, 414, 511, 512, 513, 514, 611, 612, 613, 614) of the electrode group (10, 210, 310, 410, 510, 610) at a predetermined position of the body of the user, wherein, on a right arm or a left arm, at least a first electrode is disposed correspondingly to an antebrachial musculus extensor carpi radialis brevis, a second electrode is disposed correspondingly to an antebrachial musculus flexor carpi radialis, a third electrode is disposed correspondingly to a biceps brachii muscle, and a fourth electrode is disposed correspondingly to a triceps brachii muscle, and,
under a condition that one or more electrodes among the first electrode, the second electrode, the third electrode, and the fourth electrode are each set as the first polarity and that one or more remaining electrodes are each set as the second polarity, the driving means (20, 220, 320, 420, 520, 620) is configured to selectively perform switching among the electrodes each of which is set as the first polarity and as the second polarity.

9. The electrical stimulator (1, 2, 3, 4, 5, 6) according to claim 1, further comprising a garment (40, 340, 540, 640) configured to dispose each electrode (11, 12, 13, 14, 211, 212, 213, 214, 311, 312, 313, 314, 411, 412, 413, 414, 511, 512, 513, 514, 611, 612, 613, 614) of the electrode group (10, 210, 310, 410, 510, 610) at a predetermined position of the body of the user, wherein, on a right leg or a left leg, at least a first electrode is disposed correspondingly to a biceps femoris muscle, a second electrode is disposed correspondingly to a quadriceps femoris muscle, a third electrode is disposed correspondingly to a triceps surae muscle, and a fourth electrode is disposed correspondingly to a tibialis anterior muscle, and,
under a condition that one or more electrodes among the first electrode, the second electrode, the third electrode, and the fourth electrode are each set as the first polarity and that one or more remaining electrodes are each set as the second polarity, the driving means (20, 220, 320, 420, 520, 620) is configured to selectively perform switching among the electrodes each of which is set as the first polarity and as the second polarity.

## Patentansprüche

1. Elektrischer Stimulator (1, 2, 3, 4, 5, 6), der so konfiguriert ist, dass er eine elektrische Stimulation an einen Benutzer anlegt, wobei der elektrische Stimulator (1, 2, 3, 4, 5, 6) umfasst:
eine Elektrodengruppe (10, 210, 310, 410, 510, 610), die drei oder mehr Elektroden (11, 12, 13, 14, 211, 212, 213, 214, 311, 312, 313, 314, 411, 412, 413, 414, 511, 512, 513, 514, 611, 612, 613, 614) aufweist, die so angeordnet sind, dass sie in Kontakt mit dem Benutzer kommen;
eine Ansteuereinrichtung (20, 220, 320, 420, 520, 620), die so konfiguriert ist, dass sie der Elektrodengruppe (10, 210, 310, 410, 510, 610) Energie zuführt, unter der Bedingung, dass eine oder mehrere Elektroden der Elektrodengruppe (10, 210, 310, 410, 510, 610) jeweils auf eine erste Polarität eingestellt sind und dass eine oder mehrere andere Elektroden jeweils auf eine zweite Polarität eingestellt sind, während sie ein Umschalten zwischen mindestens entweder den Elektroden, die jeweils auf die erste Polarität eingestellt sind, oder den Elektroden, die jeweils auf die zweite Polarität eingestellt sind, durchführt; und
eine Verbindungsfehler-Erfassungseinrichtung (30, 330), die einen NPN-Bipolartransistor (31, 331) mit geerdetem Emitter umfasst, wobei die Verbindungsfehler-Erfassungseinrichtung (30, 330) so konfiguriert ist, dass sie einen elektrischen Verbindungsfehler erfasst, indem sie eine der Elektroden auf die erste Polarität einstellt, während sie die anderen der Elektroden auf die zweite Polarität einstellt, um elektrisch zu erfassen, ob eine elektrische Stimulation an den Benutzer angelegt wurde, wenn der einen der Elektroden Energie zugeführt wird, und dass sie die Einstellung und Erfassung mit einer anderen der Elektroden, die auf die erste Polarität eingestellt ist, wiederholt,
wobei jede Elektrode aus einem Leiter besteht, in dem ein Poly-3,4-Ethylendioxythiophen als elektrisch leitfähiges Polymer an einer Basis aus Fasern haftet.

2. Elektrischer Stimulator (1, 2, 3, 4, 5, 6) gemäß Anspruch 1, wobei die Ansteuereinrichtung (20, 220, 320, 420, 520, 620) so konfiguriert ist, dass sie der Elektrodengruppe (10, 210, 310, 410, 510, 610) Energie zuführt, unter der Bedingung, dass eine Elektrode der Elektrodengruppe (10, 210, 310, 410, 510, 610) auf die erste Polarität eingestellt ist und dass die verbleibende Vielzahl von Elektroden jeweils auf die zweite Polarität eingestellt ist, während sie ein Umschalten zwischen den Elektroden, die jeweils auf die erste Polarität eingestellt sind, in einer vorbestimmten Reihenfolge durchführt.

3. Elektrischer Stimulator (1, 2, 3, 4, 5, 6) gemäß Anspruch 2, wobei, wenn keine elektrische Stimulation an den Benutzer angelegt ist, wenn der Elektrodengruppe (10, 210, 310, 410, 510, 610) Energie zugeführt wird, die Verbindungsfehler-Erfassungseinrichtung (30, 330) so konfiguriert ist, dass sie erfasst, dass ein elektrischer Verbindungsfehler in der auf die erste Polarität eingestellten Elektrode vorliegt.

4. Elektrischer Stimulator (1, 2, 3, 4, 5, 6) gemäß Anspruch 2, der ferner ein Kleidungsstück (40, 340, 540, 640) umfasst, das so konfiguriert ist, dass jede Elektrode (11, 12, 13, 14, 211, 212, 213, 214, 311, 312, 313, 314, 411, 412, 413, 414, 511, 512, 513, 514, 611, 612, 613, 614) der Elektrodengruppe (10, 210, 310, 410, 510, (10, 210, 310, 410, 510, 610) an einer vorbestimmten Stelle des Körpers des Benutzers angeordnet ist, wobei mindestens eine erste Elektrode entsprechend dem Musculus rectus abdominis, eine zweite Elektrode entsprechend dem rechten Musculus obliquus abdominis und eine dritte Elektrode entsprechend dem linken Musculus obliquus abdominis angeordnet ist.

5. Elektrischer Stimulator gemäß Anspruch 2, der ferner ein Kleidungsstück (40, 340, 540, 640) umfasst, das so konfiguriert ist, dass jede Elektrode (11, 12, 13, 14, 211, 212, 213, 214, 311, 312, 313, 314, 411, 412, 413, 414, 511, 512, 513, 514, 611, 612, 613, 614) der Elektrodengruppe (10, 210, 310, 410, 510, 610) an einer vorbestimmten Stelle des Körpers des Benutzers angeordnet ist, wobei mindestens eine erste Elektrode entsprechend dem rechten Musculus rectus abdominis, eine zweite Elektrode entsprechend dem linken Musculus rectus abdominis, eine dritte Elektrode entsprechend dem rechten Musculus obliquus abdominis und eine vierte Elektrode entsprechend dem linken Musculus obliquus abdominis angeordnet ist, und
die Ansteuereinrichtung (20, 220, 320, 420, 520, 620) so konfiguriert ist, dass sie ein Umschalten zwischen den auf die erste Polarität eingestellten Elektroden durchführt, so dass die erste Elektrode und die zweite Elektrode nicht nacheinander die erste Polarität annehmen.

6. Elektrischer Stimulator (1, 2, 3, 4, 5, 6) gemäß Anspruch 5, wobei die Ansteuereinrichtung (20, 220, 320, 420, 520, 620) so konfiguriert ist, dass sie ein Umschalten zwischen den Elektroden, die jeweils auf die erste Polarität eingestellt sind, in der Reihenfolge der ersten Elektrode, der vierten Elektrode, der zweiten Elektrode und der dritten Elektrode durchführt.

7. Elektrischer Stimulator (1, 2, 3, 4, 5, 6) gemäß Anspruch 1, wobei die Ansteuereinrichtung (20, 220, 320, 420, 520, 620) so konfiguriert ist, dass sie der Elektrodengruppe (10, 210, 310, 410, 510, 610) Energie zuführt, unter der Bedingung, dass eine Elektrode der Elektrodengruppe (10, 210, 310, 410, 510, 610) auf die erste Polarität eingestellt ist und dass eine andere Elektrode auf die zweite Polarität eingestellt ist, während sie abwechselnd ein Umschalten zwischen mindestens entweder den Elektroden, die jeweils auf die erste Polarität eingestellt sind, oder den Elektroden, die jeweils auf die zweite Polarität eingestellt sind, durchführt.

8. Elektrischer Stimulator (1, 2, 3, 4, 5, 6) gemäß Anspruch 1, der ferner ein Kleidungsstück (40, 340, 540, 640) umfasst, das so konfiguriert ist, dass jede Elektrode (11, 12, 13, 14, 211, 212, 213, 214, 311, 312, 313, 314, 411, 412, 413, 414, 511, 512, 513, 514, 611, 612, 613, 614) der Elektrodengruppe (10, 210, 310, 410, 510, 610) an einer vorbestimmten Stelle des Körpers des Benutzers angeordnet ist, wobei an einem rechten Arm oder einem linken Arm mindestens eine erste Elektrode entsprechend einem antebrachialen Musculus extensor carpi radialis brevis, eine zweite Elektrode entsprechend einem antebrachialen Musculus flexor carpi radialis, eine dritte Elektrode entsprechend einem Musculus biceps brachii und eine vierte Elektrode entsprechend einem Musculus triceps brachii angeordnet ist, und,
unter der Bedingung, dass eine oder mehrere Elektroden unter der ersten Elektrode, der zweiten Elektrode, der dritten Elektrode und der vierten Elektrode jeweils auf die erste Polarität eingestellt sind und dass eine oder mehrere verbleibende Elektroden jeweils auf die zweite Polarität eingestellt sind, die Ansteuereinrichtung (20, 220, 320, 420, 520, 620) so konfiguriert ist, dass sie selektiv ein Umschalten zwischen den Elektroden, die jeweils auf die erste Polarität und auf die zweite Polarität eingestellt sind, durchführt.

9. Elektrischer Stimulator (1, 2, 3, 4, 5, 6) gemäß Anspruch 1, der ferner ein Kleidungsstück (40, 340, 540, 640) umfasst, das so konfiguriert ist, dass jede Elektrode (11, 12, 13, 14, 211, 212, 213, 214, 311, 312, 313, 314, 411, 412, 413, 414, 511, 512, 513, 514, 611, 612, 613, 614) der Elektrodengruppe (10, 210, 310, 410, 510, 610) an einer vorbestimmten Stelle des Körpers des Benutzers angeordnet ist, wobei an einem rechten Bein oder einem linken Bein mindestens eine erste Elektrode entsprechend einem Musculus biceps femoris, eine zweite Elektrode entsprechend einem Musculus quadriceps femoris, eine dritte Elektrode entsprechend einem Musculus triceps surae und eine vierte Elektrode entsprechend einem Musculus tibialis anterior angeordnet ist, und,
unter der Bedingung, dass eine oder mehrere Elektroden unter der ersten Elektrode, der zweiten Elektrode, der dritten Elektrode und der vierten Elektrode jeweils auf die erste Polarität eingestellt sind und dass eine oder mehrere verbleibende Elektroden jeweils auf die zweite Polarität eingestellt sind, die Ansteuereinrichtung (20, 220, 320, 420, 520, 620) so konfiguriert ist, dass sie selektiv ein Umschalten zwischen den Elektroden, die jeweils auf die erste Polarität und auf die zweite Polarität eingestellt sind, durchführt.

## Revendications

1. Stimulateur électrique (1, 2, 3, 4, 5, 6) configuré pour appliquer une stimulation électrique à un utilisateur, le stimulateur électrique (1, 2, 3, 4, 5, 6) comprenant :
un groupe d'électrodes (10, 210, 310, 410, 510, 610) comportant trois électrodes ou plus (11, 12, 13, 14, 211, 212, 213, 214, 311, 312, 313, 314, 411, 412, 413, 414, 511, 512, 513, 514, 611, 612, 613, 614) disposées de manière à entrer en contact avec l'utilisateur ;
un moyen d'actionnement (20, 220, 320, 420, 520, 620) configuré pour fournir de l'énergie au groupe d'électrodes (10, 210, 310, 410, 510, 610), à condition qu'une ou plusieurs électrodes du groupe d'électrodes (10, 210, 310, 410, 510, 610) soient chacune réglées sur une première polarité et qu'une ou plusieurs autres électrodes soient chacune réglées sur une seconde polarité, tout en effectuant une commutation entre au moins soit les électrodes dont chacune est réglée sur la première polarité, soit les électrodes dont chacune est réglée sur la seconde polarité ; et
un moyen de détection de défaut de connexion (30, 330) comprenant un transistor bipolaire NPN à émetteur mis à la terre (31, 331), dans lequel le moyen de détection de défaut de connexion (30, 330) est configuré pour détecter un défaut de connexion électrique en réglant l'une des électrodes sur la première polarité tout en réglant les autres des électrodes sur la seconde polarité, pour détecter électriquement si une stimulation électrique a été appliquée à l'utilisateur lorsque de l'énergie est fournie à l'une des électrodes, et pour répéter le réglage et la détection avec une autre des électrodes réglée sur la première polarité,
chaque électrode étant constituée d'un conducteur dans lequel un poly-3,4-éthylènedioxythiophène en tant que polymère électroconducteur adhère à une base en fibre.

2. Stimulateur électrique (1, 2, 3, 4, 5, 6) selon la revendication 1, dans lequel le moyen d'actionnement (20, 220, 320, 420, 520, 620) est configuré pour fournir de l'énergie au groupe d'électrodes (10, 210, 310, 410, 510, 610), à condition qu'une électrode du groupe d'électrodes (10, 210, 310, 410, 510, 610) soit réglée sur la première polarité et que la pluralité restante d'électrodes soit chacune réglée sur la seconde polarité, tout en effectuant une commutation entre des électrodes dont chacune est réglée sur la première polarité dans un ordre prédéterminé.

3. Stimulateur électrique (1, 2, 3, 4, 5, 6) selon la revendication 2, dans lequel, si la stimulation électrique n'est pas appliquée à l'utilisateur lorsque de l'énergie est fournie au groupe d'électrodes (10, 210, 310, 410, 510, 610), le moyen de détection de défaut de connexion (30, 330) est configuré pour détecter qu'il y a un défaut de connexion électrique dans l'électrode réglée sur la première polarité.

4. Stimulateur électrique (1, 2, 3, 4, 5, 6) selon la revendication 2, comprenant en outre un vêtement (40, 340, 540, 640) configuré pour disposer chaque électrode (11, 12, 13, 14, 211, 212, 213, 214, 311, 312, 313, 314, 411, 412, 413, 414, 511, 512, 513, 514, 611, 612, 613, 614) du groupe d'électrodes (10, 210, 310, 410, 510, 610) à une position prédéterminée du corps de l'utilisateur, dans lequel au moins une première électrode est disposée en correspondance avec les muscles abdominaux droits, une deuxième électrode est disposée en correspondance avec les muscles abdominaux obliques droits, et une troisième électrode est disposée en correspondance avec les muscles abdominaux obliques gauches.

5. Stimulateur électrique selon la revendication 2, comprenant en outre un vêtement (40, 340, 540, 640) configuré pour disposer chaque électrode (11, 12, 13, 14, 211, 212, 213, 214, 311, 312, 313, 314, 411, 412, 413, 414, 511, 512, 513, 514, 611, 612, 613, 614) du groupe d'électrodes (10, 210, 310, 410, 510, 610) à une position prédéterminée du corps de l'utilisateur, dans lequel au moins une première électrode est disposée en correspondance avec les muscles abdominaux droits, une deuxième électrode est disposée en correspondance avec les muscles abdominaux gauches, une troisième électrode est disposée en correspondance avec les muscles abdominaux obliques droits, et une quatrième électrode est disposée en correspondance avec les muscles abdominaux obliques gauches, et
le moyen d'actionnement (20, 220, 320, 420, 520, 620) est configuré pour effectuer une commutation entre les électrodes réglées sur la première polarité de sorte que la première électrode et la deuxième électrode ne deviennent pas consécutivement la première polarité.

6. Stimulateur électrique (1, 2, 3, 4, 5, 6) selon la revendication 5, dans lequel le moyen d'actionnement (20, 220, 320, 420, 520, 620) est configuré pour effectuer une commutation entre les électrodes dont chacune est réglée sur la première polarité dans l'ordre de la première électrode, de la quatrième électrode, de la deuxième électrode et de la troisième électrode.

7. Stimulateur électrique (1, 2, 3, 4, 5, 6) selon la revendication 1, dans lequel le moyen d'actionnement (20, 220, 320, 420, 520, 620) est configuré pour fournir de l'énergie au groupe d'électrodes (10, 210, 310, 410, 510, 610), à condition qu'une électrode du groupe d'électrodes (10, 210, 310, 410, 510, 610) soit réglée sur la première polarité et qu'une autre électrode soit réglée sur la seconde polarité, tout en effectuant alternativement une commutation entre au moins les électrodes dont chacune est réglée sur la première polarité ou les électrodes dont chacune est réglée sur la seconde polarité.

8. Stimulateur électrique (1, 2, 3, 4, 5, 6) selon la revendication 1, comprenant en outre un vêtement (40, 340, 540, 640) configuré pour disposer chaque électrode (11, 12, 13, 14, 211, 212, 213, 214, 311, 312, 313, 314, 411, 412, 413, 414, 511, 512, 513, 514, 611, 612, 613, 614) du groupe d'électrodes (10, 210, 310, 410, 510, 610) à une position prédéterminée du corps de l'utilisateur, dans lequel, sur un bras droit ou un bras gauche, au moins une première électrode est disposée en correspondance avec un muscle extenseur radial court du carpe antébrachial, une deuxième électrode est disposée en correspondance avec un muscle fléchisseur radial du carpe antébrachial, une troisième électrode est disposée en correspondance avec un muscle biceps brachial, et une quatrième électrode est disposée en correspondance avec un muscle triceps brachial, et,
à condition qu'une ou plusieurs électrodes parmi la première électrode, la deuxième électrode, la troisième électrode et la quatrième électrode soient toutes réglées sur la première polarité et qu'une ou plusieurs électrodes restantes soient toutes réglées sur la seconde polarité, le moyen d'actionnement (20, 220, 320, 420, 520, 620) est configuré pour effectuer sélectivement une commutation entre les électrodes dont chacune est réglée sur la première polarité et sur la seconde polarité.

9. Stimulateur électrique (1, 2, 3, 4, 5, 6) selon la revendication 1, comprenant en outre un vêtement (40, 340, 540, 640) configuré pour disposer chaque électrode (11, 12, 13, 14, 211, 212, 213, 214, 311, 312, 313, 314, 411, 412, 413, 414, 511, 512, 513, 514, 611, 612, 613, 614) du groupe d'électrodes (10, 210, 310, 410, 510, 610) à une position prédéterminée du corps de l'utilisateur, dans lequel, sur une jambe droite ou une jambe gauche, au moins une première électrode est disposée en correspondance avec un muscle biceps fémoral, une deuxième électrode est disposée en correspondance avec un muscle quadriceps fémoral, une troisième électrode est disposée en correspondance avec un muscle triceps sural, et une quatrième électrode est disposée en correspondance avec un muscle tibial antérieur, et,
à condition qu'une ou plusieurs électrodes parmi la première électrode, la deuxième électrode, la troisième électrode et la quatrième électrode soient toutes réglées sur la première polarité et qu'une ou plusieurs électrodes restantes soient toutes réglées sur la seconde polarité, le moyen d'actionnement (20, 220, 320, 420, 520, 620) est configuré pour effectuer sélectivement une commutation entre les électrodes dont chacune est réglée sur la première polarité et sur la seconde polarité.
